(19) Europäisches Patentamt
European Patent Office
Office européen des brevets

(11) **EP 1 811 294 A1**

(12) **EUROPEAN PATENT APPLICATION**
published in accordance with Art. 158(3) EPC

(43) Date of publication:
**25.07.2007 Bulletin 2007/30**

(21) Application number: **05788275.5**

(22) Date of filing: **30.09.2005**

(51) Int Cl.:
***G01N 27/62*** *(2006.01)* ***G01N 33/483*** *(2006.01)*

(86) International application number:
**PCT/JP2005/018134**

(87) International publication number:
**WO 2006/043405 (27.04.2006 Gazette 2006/17)**

(84) Designated Contracting States:
**DE GB**

(30) Priority: **18.10.2004 JP 2004303358**

(71) Applicant: **Mitsubishi Chemical Corporation**
**Minato-ku**
**Tokyo 108-0014 (JP)**

(72) Inventors:
- **KANIE, Osamu c/o MITSUBISHI KAGAKU INSTITUTE OF**
  **Tokyo 1948511 (JP)**
- **KURIMOTO, Ayako c/o MITSUBISHI KAGAKU INSTITUTE**
  **Tokyo 1948511 (JP)**
- **DAIKOKU, Syusaku c/o MITSUBISHI KAGAKU INSTITUTE**
  **Tokyo 1948511 (JP)**

(74) Representative: **polypatent**
**An den Gärten 7**
**51491 Overath (DE)**

(54) **METHOD OF ANALYZING STRUCTURE OF SUGAR CHAIN**

(57) It is an objective of the present invention to provide a method of analyzing the structure of a sugar chain by performing CID-$MS^n$ measurement of a sugar chain of unknown structure and comparing the obtained data with obtained reference data acquired in advance.

Fig.1

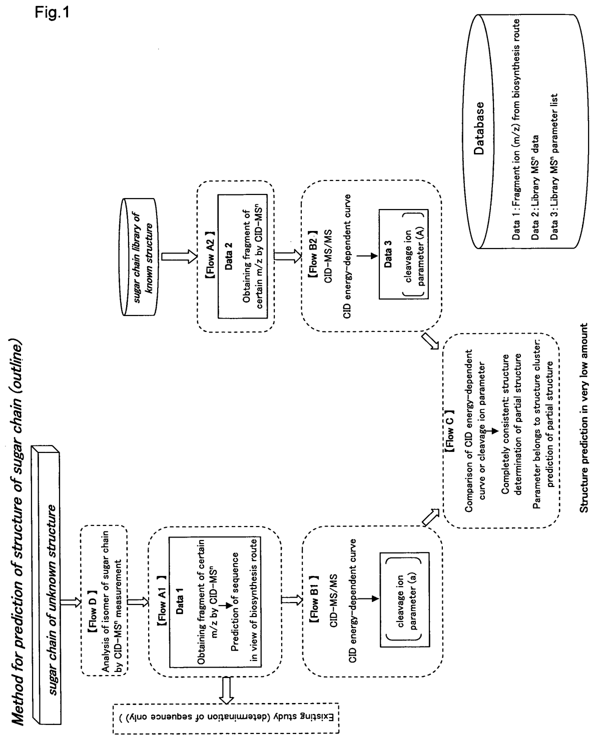


EP 1 811 294 A1

## Description

Technical Field

The present invention relates to a method of analyzing the structure of a sugar chain, comprising performing a CID-$MS^n$ measurement of a sugar chain of unknown structure and comparing the obtained data with reference data acquired in advance. Further, the present invention relates to a CID energy-dependent curve library comprising CID energy-dependent curves showing the relationships between the CID energies and the total ion counts of a plurality of daughter ions each having a specific m/z that are obtained via CID-$MS^n$ measurement of a plurality of reference sugar chains of known structures.

Background Art

Sugar chains mainly exist as parts of glycoproteins or glycolipids on cell surfaces. Sugar chains are deeply involved in a variety of life phenomena related to development, differentiation induction, fertilization, immunization, oncogenic transformation, infectious diseases, and the like. In recent years, studies of sugar chains having various functions as described above have been gaining momentum. In addition, sugar chains play important roles in protein posttranslational modification and thus they will remain important subjects of study for the near future. Functions of glycoproteins or glycolipids are controlled by sugar chains in some cases. Therefore, a technology whereby structural analysis of a minute amount of a sugar chain is achieved is required. However, sugar chains belong to a molecular species that cannot be amplified by bioengineering techniques. Accordingly, it is necessary to develop a new technology for structural analysis that allows the structural analysis to be achieved with the use of a minute amount of a substance alone. In the cases of protein sequences, if their genomic sequences are known, protein amino acid sequences corresponding thereto can be obtained. Thus, the amino acid sequence of a target protein can be analyzed by mass spectrometry (e.g., the MS/MS method). However, technology for analyzing modified protein sequences has remained a challenge for the future. In particular, protein modification with the use of sugar chains results in a diversity of very large molecules. Thus, development of a structural analysis technology for sugar chains is indispensable.

Sugar chains, unlike nucleic acids and proteins, form a group of structural isomers due to factors other than sequences. Basically, this is because monosaccharides forming a sugar chain have a plurality of hydroxyl groups that serve as reaction points so that sugar chains have properties of forming bond positional isomers and forming anomeric isomers due to anomer isomerism in the case of binding between monosaccharides. The binding between monosaccharides is carried out *in vivo* by a continuous reaction of a group of enzymes that are involved in sugar chain synthesis. Thus, a group of sugar chains is not necessarily formed based on the principles of combination therebetween. However, enzyme reactions are known to accompany side reactions. Also, the significance of such phenomena found in organisms has not been elucidated. In such cases, a structural analysis method based on a totally new concept without the use of genetic information is required. Needless to say, a new structural analysis method is required for sugar chain structural analysis in the case of an organism species for which genetic information and the biosynthetic routes of sugar chains have not been elucidated.

Examples of technologies for sugar chain structural analysis that have been recently used include (1) nuclear magnetic resonance spectroscopy, (2) mass spectrometry, (3) a mapping method via multidimensional chromatography (see Royle, L. et al., Anal. Biochem., 2002, 304, 70-90), (4) a specific partial hydrolysis method with the use of hydrolase (see Chaplin, M. F. et al., Carbohydrate Analysis, A Practical Approach, IRL Press, Oxford, 1994), (5) a mapping method with the use of a lectin (see Chaplin, M.F et al., Carbohydrate Analysis, A Practical Approach, IRL Press, Oxford, 1994), and (6) a composition analysis method via methylation-hydrolysis-gas chromatography (see Chaplin, M. F et al., Carbohydrate Analysis, A Practical Approach, IRL Press, Oxford, 1994). In general, these examples are used in combination for sugar chain analysis.

Nuclear magnetic resonance spectroscopy and mass spectrometry are very efficient techniques for sugar chain analysis among the above examples. The drawback of the former is that a necessary amount of a sample subjected to analysis is at least on the microgram-order. In addition, the drawback of the latter is that stereoisomer analysis cannot be carried out. With the use of any of the above analysis methods, or similar methods, it is necessary for a naturally occurring sugar chain obtained to be directly subjected to structural analysis (see Dell, A., Adv. Carbohydr. Chem. Biochem. 1987, 45, 19-72).

Meanwhile, it has been shown that mass spectrometry of different sugar chains results in the obtaining of different results. For instance, with regard to fragmentation of stereoisomers of hydroxyl groups of a steroid via CID-MS/MS measurement, it has been shown that different fragment strengths are obtained (see Faretto, D. et al., Mass Spectrom., 1991, 5, 240-244), and that different values are obtained by allowing structural isomers of a sulfonated sugar chain that has been synthesized to be subjected to CID-MS/MS measurement via FAB-q-Mass and measuring the strengths of specific ions (see Kurono, S. et al., J. Mass Spectrom., 1998, 33, 35-44). However, a specific method of analyzing the structure of a sugar chain has not been derived from any of these facts.

Concerning a structural analysis technology for sugar chains with the use of a mass spectrometry apparatus, a general structural analysis method is a method wherein an m/z derived from a sugar chain and the peak intensity thereof are designated as parameters and such parameters are compared with those of a sugar chain of known structure, so that it becomes possible to determine whether or not the structures of the two sugar chains are identical to each other (see Viseux. N. et al., Anal. Chem. 1998, 70, 4951-4959; and Kameyama, A. et al., Anal. Chem. 2005, 77, 4719-4725). In accordance with such method, the peak intensities of fragments at a specific voltage are designated as indices and the strengths of the fragments are scored for determining whether or not the scores are comprehensively consistent with each other. Thus, an exhaustive data set or database of a group of reference compounds is needed. In addition, the limitation of such method is that the method is merely used for determination of consistency and thus it is impossible to carry out structural prediction in a case in which there is no consistency between the scores.

In the case of a group of compounds that are structurally similar to each other, there has been a report suggesting that multivariate analysis leads to an alternative possibility of extracting structural features from data obtained by mass spectrometry. However, such report merely suggests that such analysis can be applied to structural prediction of disaccharides. Nothing has been specifically mentioned therein regarding the possibility of predicting the structure of a long sugar chain (Faengmark, I. et al., Anal. Chem. 1999, 71, 1105-1110).

Further, mass spectrometry is a type of super microanalysis. Thus, by mass spectrometry, it is possible to know the molecular weight of a substance based on the detected mass number (e.g., addition of H, Na, or the like) and to obtain important information regarding molecular structures from cleavage forms of fragment ions. However, in a case in which a sample contains an isomer (e.g. structural isomer or an optical isomer), it is impossible to determine even the existence of such a contaminant with the exclusive use of a mass spectrometer.

Disclosure of the Invention

Object to be Solved by the Invention

It is an objective of the present invention to provide a method of analyzing the structure of a sugar chain by performing CID-$MS^n$ measurement of a sugar chain of unknown structure and comparing the obtained data with obtained reference data acquired in advance. Specifically, it is an objective of the present invention to provide a method of determining the structure of a sugar chain by performing CID-MS/MS measurement of a fragment ion having a specific m/z of a target sugar chain for structural analysis, comparing the obtained CID energy-dependent curve with a CID energy-dependent curve contained in reference data relating to a daughter ion having the same m/z that has been obtained from a parent ion having the same m/z, and determining the consistency therebetween. It is another objective of the present invention to provide a method of determining or predicting the structure of a target sugar chain comprising creating cleavage ion parameters shown in numerical values that characterize CID energy-dependent curves of the target sugar chain and a reference sugar chain, respectively, and statistically analyzing the parameters. It is another objective of the present invention to provide an analysis method for identifying a mixture of structural isomers of a sugar chain with the exclusive use of a mass spectrometer.

Means for Solving the Object

In order to achieve the above objectives, the present inventors have made intensive studies. They synthesized sugar chains having different structures and stereoisomeric sugar chains and performed CID-$MS^n$ measurement until fragment ions each having a specific m/z were obtained. They further performed CID-MS/MS measurement of the fragment ions and created CID energy-dependent curves each showing the relationship between the CID energy and the total ion count of the obtained daughter ion having a specific m/z. Accordingly, they have found that identical CID energy-dependent curves can be obtained by repeatedly measuring sugar chains having identical structures, while on the other hand, different CID energy-dependent curves can be obtained in the cases of sugar chains having different structures. In addition, they have found that different CID energy-dependent curves can be obtained from stereoisomers. Further, they have found that a partial structure of a sugar chain can be predicted based on statistical analysis of parameters, even in a case in which there is no consistency in terms of partial structures of sugar chains, CID energy-dependent curves obtained therefrom, or parameters of such CID energy-dependent curves. The present invention has been completed based on the above findings.

That is, in accordance with the present invention, the following inventions are provided.

(1) A method of analyzing the structure of a sugar chain, comprising: (a) performing CID-$MS^n$ measurement of a target sugar chain until a fragment ion having a specific m/z is obtained; (b) further performing CID-MS/MS measurement of the fragment ion and creating a CID energy-dependent curve that shows the relationship between the CID energy and the total ion count of the obtained daughter ion having a specific m/z; and (c) comparing the CID energy-dependent curve with a CID energy-dependent curve of a daughter ion having an m/z identical to that of the above daughter ion, which is obtained via CID-MS/MS measurement of a fragment ion that has an m/z identical to that of the above fragment ion and is obtained from a reference sugar chain of known structure.

(2) A CID energy-dependent curve library comprising CID energy-dependent curves showing the relationships between the CID energies and the total ion counts of a plurality of daughter ions each having a specific m/z, which are obtained via CID-$MS^n$ measurement of a plurality of reference sugar chains of known structures.

(3) The library according to (2), wherein each reference sugar chain comprises trisaccharide.

(4) A cleavage ion parameter library comprising cleavage ion parameters of a plurality of reference sugar chains of known structures, which is created from the CID energy-dependent curves according to (2).

(5) The library according to (4), wherein the cleavage ion parameters of a plurality of reference sugar chains of known structures comprise at least one parameter selected from the group consisting of a relative value represented by the maximum value of ionic strength of a daughter ion having a specific m/z with respect to the total ion count of the daughter ion, a CID energy value at which 50% of the above relative value is obtained, and the slope at the inflection point of the CID energy-dependent curve.

(6) The library according to (4) or (5), wherein each reference sugar chain comprises trisaccharide.

(7) A method of analyzing the structure of a sugar chain, comprising: (a) performing CID_$MS^n$ measurement of a target sugar chain until a fragment ion having a specific m/z is obtained; (b) further performing CID-MS/MS measurement of the fragment ion and creating a CID energy-dependent curve showing the relationship between the CID energy and the total ion count of the obtained daughter ion having a specific m/z; and (c) comparing the CID energy-dependent curve with a CID energy-dependent curve of a daughter ion having an m/z identical to that of the above daughter ion, which is obtained by performing CID-MS/MS measurement of a fragment ion having an m/z identical to that of the above fragment ion contained in the library of (2) or (3).

(8) The method according to (1) or (7), comprising creating cleavage ion parameters of a CID energy-dependent curve of a daughter ion having a specific m/z that is obtained from a target sugar chain in step (c) according to (1) or (7) and comparing the cleavage ion parameters with cleavage ion parameters of a CID energy-dependent curve of a daughter ion having an m/z identical to that of the above daughter ion of the reference sugar chain of known structure.

(9) The method according to (8), wherein cleavage ion parameters comprise at least one parameter selected from the group consisting of a relative value represented by the maximum value of ionic strength of a daughter ion having a specific m/z that is relative to the total ion count of the daughter ion, a CID energy value at which 50% of the above relative value is obtained; and the slope at the inflection point of the CID energy-dependent curve.

(10) A method of analyzing the structure of a sugar chain, comprising: (a) performing CID-$MS^n$ measurement of a target sugar chain until a fragment ion having a specific m/z is obtained; (b) further performing CID-MS/MS measurement of the fragment ion and creating a CID energy-dependent curve showing the relationship between the CID energy and the total ion count of the obtained daughter ion having a specific m/z; (c) creating cleavage ion parameters of the CID energy-dependent curve; (d) comparing the cleavage ion parameters with cleavage ion parameters created from a CID energy-dependent curve of a daughter ion having an m/z identical to that of the above daughter ion, which is obtained via CID-MS/MS measurement of a fragment ion having an m/z identical to that of the above fragment ion contained in a library according to (4) to (6).

(11) The method according to (1) or any one of (7) to (10), wherein the total ion count of the daughter ion having a specific m/z, from which a CID energy-dependent curve is obtained, is represented by the proportion of the total ion count of the daughter ion having a specific m/z to the sum of the total ion count of such daughter ion and the total ion count of the parent ion.

(12) A method of analyzing the structure of a sugar chain, comprising allowing fragment ions having different specific m/z levels contained in a target sugar chain to be repeatedly subjected to the steps (a) to (c) in (1) or (7) or the steps (a) to (d) in (10) and combining the obtained results.

(13) A system for analyzing the structure of a sugar chain, comprising at least a CID-$MS^n$ measurement apparatus, a recording medium on which the library according to any one of (2) to (6) is recorded so as to be readable by a computer, and a recording medium on which a program used for allowing different specific fragment ions contained in a target sugar chain to be subjected to steps (b) and (c) in (1) or (7) or the steps (b) to (d) in (10) is recorded.

(14) A method of examining contamination of a sugar chain structural isomer, comprising: (a) performing CID-$MS^n$ measurement of a sugar chain test sample at a given voltage at which a fragment ion having a specific m/z does not disappear; (b) further performing CID-MS/MS measurement of the remaining fragment ion; and (c) comparing mass spectra, CID energy-dependent curves, or cleavage ion parameters of such curves, which are obtained in the above two steps, with each other.

Best Mode for Carrying Out the Invention

The present invention is hereafter described in greater detail. The following descriptions of constituent features of the present invention are representative examples of the embodiments of the present invention. Thus, the technical scope of the present invention is not limited thereto.

In descriptions below, the term "CID-$MS^n$ measurement" refers to the obtaining of multistage mass spectrometry by CID (collision induced dissociation). The term "fragment ion" indicates an ion having an m/z, which is obtained by the above CID-$MS^n$ measurement, physicochemical degradation, or the like. The term "m/z" indicates the proportion of mass number (m) to electric charge (z). The term "daughter ion" indicates a fragment ion having an m/z obtained by allowing such fragment ion to be subjected to the above CID-$MS^n$ measurement. The term "total ion count" indicates a sum of ionic strengths of all fragment ions each having an m/z. Also, in general, the term "CID energy" indicates energy that is added upon CID, and it refers to a voltage of an electric field of a certain frequency that causes ion vibration in practice. Further, the term "method of analyzing the structure" indicates a method of determining and/or predicting a structure.

The method of the present invention is described in figs. 1 to 5. The present invention will be described as follows with reference to the figures,.

(1) The outline of the structural analysis method (fig. 1)

In accordance with the present invention, a sugar chain of unknown structure (hereafter referred to as "target sugar chain") is subjected to CID-$MS^n$ measurement until a fragment ion (hereafter referred to as "parent ion" or "parent fragment ion") of a specific m/z is obtained (flow A1), and the obtained parent fragment ion is then further subjected to CID-MS/MS measurement (flow B1). Then, a CID energy-dependent curve showing the relationship between the CID energy and the total ion count of the obtained daughter ion having a specific m/z is created. If necessary, cleavage ion parameters that indicate respective properties of the curve are produced (flow B1). Meanwhile, a sugar chain of known structure (hereafter referred to as a "reference sugar chain" in some cases) or a library thereof serving as reference data is subjected to CID-$MS^n$ measurement until a fragment ion of a specific m/z is obtained (flow A2). Then, the obtained fragment ion is further subjected to CID-MS/MS measurement (flow B2). Then, a CID energy-dependent curve showing the relationship between the CID energy and the total ion count of a specific daughter ion having is created. If necessary, cleavage ion parameters that indicate respective properties of the curve are produced (flow B2). Herein, such CID energy-dependent curve and such cleavage ion parameter of a reference sugar chain are collectively referred to as "reference data" in some cases. In addition, a test of examining contamination of a sugar chain structural isomer in a target sugar chain (flow D) may be carried out by CID-$MS^n$ measurement prior to flow Al.

The thus obtained CID energy-dependent curve or cleavage ion parameter derived from a daughter ion of a specific m/z that is obtained from a fragment ion of a specific m/z of a target sugar chain is compared with that of reference data (flow C). If there is reference data that corresponds to data relating to the target sugar chain, it is possible to determine that a partial structure of the reference sugar chain from which the reference data is obtained corresponds to that of the target sugar chain.

In addition, even in a case in which reference data does not correspond to data relating to the target sugar chain, the partial structure can be predicted as long as parameters of the target sugar chain fall within the range of statistical analysis results relating to parameters of the reference sugar chain. The present invention encompasses a method for such structural prediction.

When a partial structure of a target sugar chain is determined or predicted as described above, the entire structure thereof can be determined or predicted by, for example, referring to the biosynthetic route of the target sugar chain (flow A1).

(2) Preparation of a target parent fragment ion (fig. 1, fig 2/flow A1)

A target sugar chain used in the present invention may be any sugar chain with the proviso that it be possible to subject it to, for example, degradation by methods described below so that it can then be subjected to CID-$MS^n$ measurement. In addition, such target sugar chain is subjected to the method of the present invention in order to analyze the structure thereof. Thus, it is preferable that the whole or a partial structure thereof be unknown. Such target sugar chain may be obtained from tissue of a living body, cells, or a sugar chain bound to a synthesized protein. Further, such target sugar chain may be subjected to acid hydrolysis or enzyme degradation, followed by separation and purification by HPLC or the like before use. Moreover, a chemically synthesized sugar chain can be used as such target sugar chain.

In accordance with the present invention, daughter ions are obtained by CID-$MS^n$ measurement of parent ions having an identical m/z that are obtained from a target sugar chain and a reference sugar chain, and ion strengths of the obtained daughter ions are compared. Thus, it is necessary to carry out degradation by a given method until a fragment ion (hereafter referred to as a "target parent fragment ion") having an m/z identical to that of a parent ion (hereafter referred to as a "reference parent fragment ion") from which reference data is derived is obtained. Conventional degradation methods that are known to the public can be used. For instance, a sugar chain obtained by acid hydrolysis, enzyme degradation, or the like can be used after being separated and purified by liquid chromatography, capillary electrophoresis, gel electrophoresis, or the like. Alternatively, an arbitrary fragment ion obtained by CID-MS$^n$ measurement or the like described below can be used after being separated based on m/z. Flow Al shows an example in which a trisaccharide library is used as a reference sugar chain. In such case, it is preferable to degrade a target sugar chain into mono-, di-, or tri-saccharides. In addition, when a target sugar chain has a substituent, such substituent may be removed. Also, if there is a sugar chain having a substituent in reference data, such sugar chain may be used in such condition. Degraded products of the target sugar chain obtained as final products are separately subjected to mass spectrometry for confirmation of m/z. Sequencing can be carried out based on the thus obtained mass and other forms of information such as a biosynthetic route. However, the method of the present invention can be applied for a fragment ion that cannot be determined in the manner described above.

(3) CID-MS$^n$ measurement of a target parent fragment ion (fig. 1, fig. 3/flow B1)

The target parent fragment ions obtained above are subjected to CID-MS$^n$ measurement. (A case in which a target parent fragment obtained by CID-MS$^n$ measurement is subjected to CID-MS/MS measurement.)

As an ionization method, FAB (fast atom bombardment method), CI (chemical ionization), ESI (electro-spray ionization method), MALDI (matrix assisted laser desorption/ionization), APCI (atmospheric pressure chemical ionization), and the like are used. In accordance with the present invention, the ESI method is preferably used in terms of the ease of sample preparation and no influence of a matrix-derived contaminant ion. However, as long as the above CID-MS$^n$ measurement can be carried out, the ionization method is not limited thereto. In addition, the ESI method includes a micro spray method and a nano-spray method. In view of the amount of a sample used, a nano-spray method is preferably used in the present invention.

When the above target sugar chain or a degraded product thereof is ionized, a minute amount of a solution is ionized by evaporating a solvent *in vacuo.* Thus, water/methanol or water/acetonitrile (1:1) can be used as a solvent. However, such solvent can be selected depending on properties of a substance and thus is not limited to such examples. A target sugar chain or a degraded product thereof is dissolved into such solvent to a concentration of 0.01 to 100 nmol/ml, preferably 0.5 to 5 nmol/ml, and more preferably 1 nmol/m1.

In addition, any apparatus for CID-MS$^n$ measurement can be used as long as it can carry out CID-MS$^n$ measurement. However, in accordance with the present invention, a quadrupole ion trap mass spectrometer capable of carrying out CID-MS$^n$ measurement is preferably used. Specific examples of such apparatus include esquire 3000 plus (Bruker Daltonics Corp.), LCQ DECA (Thermo Finnigan), AXIMA QIT (Shimadzu Corporation), and LCMS-IT-TOF (Shimadzu Corporation). Any measurement method can be used as long as the ionic strengths of daughter ions that dissociate from each parent fragment ion can be measured. Specifically, for example, measurement can be carried out in a positive or negative mode at a thermocapillary temperature of 20˚C to 365˚C, a capillary voltage of 0 to 6.0 kV, and a CID energy of 0 to 25 V. When a positive mode is used, positive ions such as protons, sodium, potassium, lithium, calcium, and ammonium can be selected and used.

The target parent fragment ions are further subjected to the aforementioned CID-MS$^n$ measurement (or CID-MS/MS measurement). A CID energy-dependent curve showing the relationship between the CID energy and the total ion count of a daughter ion having a specific m/z is created from plots obtained via the aforementioned measurement. (equation 1). In addition, the total ion count of the daughter ion having a specific m/z is preferably represented by the proportion of the total ion count of the daughter ion to the sum of the total ion count of the daughter ion and the total ion count of the above parent ion (equation 2). Also, equations 1 and 2 are relational equations in a case in which daughter ions a, b, c, d, ..., (i), ...n are generated from a parent ion A. The daughter ion strength is represented by such proportion so that an accurate CID energy-dependent curve can be created without the influence of the ion counts of other weak ions. Such daughter ions each having a specific m/z may be any of or all of the daughter ions that disassociate from a target parent fragment ion.

[Equation 1]

$$y = \frac{[\mathrm{I.C.}^{i}]}{[\mathrm{I.C.}^{A}] + \sum_{a}^{n} [\mathrm{I.C.}^{i}]} \times 100$$

[Equation 2]

$$y = \frac{[\mathrm{I.C.}^{i}]}{[\mathrm{I.C.}^{A}] + [\mathrm{I.C.}^{i}]} \times 100$$

Further, the above CID energy-dependent curve is represented by numerical values indicating features of the curve with the use of a known method such that cleavage ion parameters can be created.

As such cleavage ion parameter or parameters, it is possible to use at least one parameter selected from the group consisting of a relative value represented by the maximum value of ionic strength of a daughter ion having a specific m/z that is relative to the total ion count of the daughter ion, a CID energy value at which 50% of the above relative value is obtained, and the slope at the inflection point of the CID energy-dependent curve. In addition, a CID energy-dependent curve is calculated by the following equation 3 with the use of the following indices: the fiducial value (Bottom = 0) and the maximum value (Top = 100) as relative values of the ion count; a CID energy ($V_{50}$) that corresponds to 50% of the difference between the relative values; and the slope at the inflection point of the CID energy-dependent curve (Slope). Thus, cleavage ion parameters can be obtained. In the equation 3, "x" indicates CID energy.

[Equation 3]

$$y = Bottom + (Top + Bottom)(1 + \exp^{\frac{V50 - x}{Slope}})$$

Upon CID-MS$^n$ measurement for creating the aforementioned CID energy-dependent curve or cleavage ion parameters, if there are data fluctuations depending on the type of CID-MS$^n$ measurement apparatus or an individual sample, it is difficult to compare data with reference data described below in an accurate manner. Thus, it is preferable that a substance that can be a standard substance also be subjected to similar CID-MS$^n$ measurement according to need and that the related data thereof be used for correction for comparison.

(4) Preparation of a reference parent fragment ion (fig. 1, fig 2/flow A2)

For the purpose of obtaining reference data, parent fragment ions of a reference sugar chain are prepared in the manner described in (2) above. Any reference sugar chain can be used as long as the whole or a partial structure of a target sugar chain can be determined by the method of the present invention. Specifically, such reference sugar chain used is likely to have a known structure that corresponds to a partial or the whole structure of a target sugar chain. Such reference sugar chain may be a naturally occurring sugar chain or a synthesized sugar chain. In addition, such reference sugar chain may have a substituent. The length of such sugar chain used is 1 to 13 sugars that are ligated with each other.

With the use of a library of a group of specific sugars as such reference sugar chain, it is possible to carry out structure determination of a target sugar chain in a exhaustive and systematic manner. Such library has a structure of, for example, 2 to 25 sugars that are ligated with each other. For instance, sugar chains constituting such library, which are preferably used, are sugar chains having sequences comprising combinations (permutations) of all types of monosaccharides that constitute naturally occurring sugar chains and stereoisomers of the same. In addition, such sugar chain may contain nonnatural monosaccharide.

Further, when a sugar chain structure is generalized, a sugar chain can be a polymer sugar compound comprising a nonreducing end sugar, a reducing end sugar, and a sugar that exists therebetween. In such case, the minimum unit of such polymer is a sugar chain in which three saccharides are ligated with each other. A trisaccharide library contains sugar chains having sequences comprising combinations (permutations) of all types of monosaccharides that constitute naturally occurring sugar chains comprising trisaccharides and stereoisomers of the same. Since such library contains partial structures of sugar chains having any linear chain structures that exist *in vivo,* it is preferable as a library of reference sugar chains of the present invention.

Examples of monosaccharides constituting the above naturally occurring sugar chains include glucose, galactose, mannose, N-acetylglucosamine, N-acetylgalactosamine, xylose, sialic acid, glucuronic acid, and fucose. In addition, such monosaccharide may have a modified group.

It is preferable to chemically synthesize a reference sugar chain and a reference sugar chain library. It is possible to use known synthesis methods that are generally used. Upon chemical synthesis, a synthetic unit in which each monosaccharide described above is appropriately protected is needed. Further, in order to synthesize a sugar chain library comprising stereoisomers with good efficiency, it is necessary to use a protecting group without neighboring group participation, such as a benzyl group (Bn), so as to obtain $\alpha$-glycoside and to use a protecting group with neighboring group participation, such as acetyl group, so as to obtain $\beta$-glycoside, upon structure-selective synthesis. For the control of such structures, it is necessary to use a protecting group in addition to the above protecting group throughout synthesis. Thus, the number of monosaccharide synthetic units increases, resulting in the complicated synthetic route. Therefore, it is preferably to simplify the synthetic route in accordance with, for example, the method of PCT/JP2004/009523 in a manner such that a 2-position protecting group is determined to be a protecting group without neighboring group participation.

The thus obtained reference sugar chain or reference sugar chain library is subjected to CID-$MS^n$ measurement as with the case of the target sugar chain until a fragment ion having a specific m/z is obtained. The term "fragment ion having a specific m/z" indicates any fragment ion having an m/z that is identical to that of a parent fragment ion of a target sugar chain. In addition, when a reference sugar chain is in the form of a library, any of or all of the fragment ions that can be cleaved may be used, such fragment ions being obtained from each constituent of the library.

(5) CID-$MS^n$ measurement of a reference parent fragment ion (fig. 1, fig. 3/flow B2)

The reference parent fragment ion obtained in (4) above is subjected to CID-$MS^n$ measurement as with the case of the target parent fragment ion. (A reference parent fragment obtained by CID-$MS^n$ measurement is subjected to CID-MS/MS measurement.) An ionization method, a solvent, a sugar chain preparation method, a measurement apparatus, and the like are all used as with the cases of the target sugar chain and the parent fragment ion. A CID energy-dependent curve showing the relationship between the CID energy and the total ion count of a daughter ion having a specific m/z is created from plots obtained via the aforementioned measurement (equation 1 above). In addition, the total ion count of the daughter ion having a specific m/z is preferably represented by the proportion of the total ion count of the daughter ion to the sum of the total ion count of the daughter ion and the total ion count of the above parent ion (equation 2 above). The daughter ion strength is represented by such proportion so that an accurate CID energy-dependent curve can be created without the influence of the ion counts of other weak ions. Daughter ions each having a specific m/z may include any of or all of the daughter ions that disassociate from a reference parent fragment ion. Further, when a reference sugar chain is in the form of a library, such daughter ions may preferably be all daughter ions that are derived from reference parent fragment ions obtained from all the constituents of the library upon exhaustive and systemic analysis.

Further, when the above CID energy-dependent curve is represented by numerical values indicating features of the curve by a known method, cleavage ion parameters can be created.

Such cleavage ion parameters that can be used comprise at least one parameter selected from the group consisting of a relative value represented by the maximum value of ionic strength of a daughter ion having a specific m/z that is relative to the total ion count of the daughter ion, a CID energy value at which 50% of the above relative value is obtained, and the slope at the inflection point of the CID energy-dependent curve. In addition, a CID energy-dependent curve is calculated by the following equation 3 with the use of the following indices: the fiducial value (Bottom = 0) and the maximum value (Top = 100) as relative values of the ion count; CID energy ($V_{50}$) that corresponds to 50% of the difference between the relative values; and the slope at the inflection point of the CID energy-dependent curve (Slope). Thus, cleavage ion parameters can be obtained. In such case, curve fitting of a CID energy-dependent curve also can be carried out by another equation so that corresponding parameters can be used.

The present invention encompasses a CID energy-dependent curve library comprising a CID energy-dependent curve showing the relationships of the CID energies and the total ion counts of a plurality of daughter ions each having a specific m/z obtained via CID-$MS^n$ measurement of a plurality of reference sugar chains of known structures, and a cleavage ion parameter library comprising cleavage ion parameters of a plurality of reference sugar chain of known structure, such cleavage ion parameters being created from the above CID energy-dependent curve.

Further, the present invention also encompasses a data set in which reference data is stored in a memory device that is readable by a computer, such reference data including a CID energy-dependent curve, cleavage ion parameters, and the like, which are obtained by CID-$MS^n$ measurement of a reference sugar chain or a reference sugar chain library.

Furthermore, a CID energy-dependent curve or a cleavage ion parameter can be used for a new type of an extendable barcode. Such barcode can be used for describing a molecular structure. In addition to reference data, the name and the origin of a reference sugar chain, the method of and conditions for measurement, and the like can be encoded into a barcode. Such barcode can be applied to a sample bottle or the like. Thus, persons skilled in the art would be able to create a barcode reader whereby such barcode can be read and related analysis software. Thus, the present invention also encompasses such barcode reader and software.

(6) Structure determination of a target sugar chain (fig. 1, fig. 4/flow C)

In accordance with the present invention, a CID energy-dependent curve of a daughter ion (hereafter referred to as a "target daughter ion" in some cases) having a specific m/z that is obtained from a parent fragment ion having a specific m/z of the target sugar chain obtained in (3) above is compared with a selected CID energy-dependent curve of a daughter ion (hereafter referred to as a "reference daughter ion") having an m/z that is identical to that of the target daughter ion, such daughter ion being obtained from a reference parent fragment ion having an m/z that is identical to that of the target parent ion. In addition, cleavage ion parameters obtained from these CID energy-dependent curves may be compared with each other.

In addition, when such reference sugar chain is in the form of a library, data relating to a parent ion having an m/z identical to that of a target parent fragment ion is first selected from reference data. Further, a CID energy-dependent curve or a cleavage ion parameter of a daughter ion having an m/z that is identical to that of the target daughter ion to be compared is selected from the data for comparison.

When a CID energy-dependent curve or a cleavage ion parameter obtained from a target parent fragment ion is consistent with that of corresponding reference data, it is possible to determine that the structure of a reference parent fragment ion from which the reference data has been derived is identical to the structure of the target parent fragment ion. Herein, data consistency means consistency between curves of 95% or more in the case of CID energy-dependent curves. Also, in the case of cleavage ion parameters, consistency is represented by a difference between parameters of within $\pm 2\%$. Further, when a substituent is bound to a target parent fragment ion, the data relating to the target parent fragment ion is compared with the reference data obtained from a reference parent fragment ion to which a substituent is bound. In such case, if the data relating to the target parent fragment ion is consistent with that relating to the reference parent fragment ion, it is possible to determine that the target parent fragment ion has a substituent that is identical to that of the reference parent fragment ion. In such case, determination can also be carried out based on a Z-test.

Even if data consistency is observed in the case of a CID energy-dependent curve or a cleavage ion parameter of a daughter ion having a specific m/z, data consistency might not be achieved when the data relating to a daughter ion having another m/z is subjected to comparison. Thus, it is preferable that a plurality of daughter ions is subjected to data comparison.

In a case in which a portion of a target sugar chain is determined by the method of the present invention, partial sugar chains that are used in the biosynthetic route are referred to and ligated with each other in order to determine the total sequence of the target sugar chain. Alternatively, in a case in which a sugar chain is first degraded in order to produce a target parent fragment ion, a method wherein the sugar chain is reconstructed based on the method for degradation is used.

(7) Structural prediction of a target sugar chain (fig. 1, fig. 4/flow C)

When data consistency is not observed after comparing the CID energy-dependent curves or cleavage ion parameters with each other in (6) above, it is possible to predict the structure of a target sugar chain by comparison between cleavage ion parameters. Specifically, cleavage ion parameters are created from a CID energy-dependent curve of a target daughter ion so as to be compared with cleavage ion parameters created from a CID energy-dependent curve of a reference daughter ion, such that it becomes possible to predict the structure of the target sugar chain. Preferably, such comparison is carried out by statistical analysis. In addition, the aforementioned sugar chain library is preferably used as a reference sugar chain. Further, a CID energy-dependent curve is subjected to calculation of the following equation 3 with the use of the following indices: the fiducial value (Bottom = 0) and the maximum value (Top = 100) as relative values of the ion count; a CID energy ($V_{50}$) that corresponds to 50% of the difference between the relative values; and the slope at the inflection point of the CID energy-dependent curve (Slope). Thus, cleavage ion parameters can be obtained.

(8) Purity test of a sugar chain structural isomer (fig. 1, fig. 5/flow D)

As described above, each sugar chain structural isomer gives rise to a different result of CID-$MS^n$ measurement (fig. 10). Specifically, sugar chain isomers having different structures differ from each other in terms of the reaction of cleavage of bonds in each structure even if such isomers have the same mass number. This is because there is a difference in terms of the chemical bond energy necessary for the formation of a structure isomer so that different bonds dissociate under different CID conditions. Based on such reason, collision-induced dissociation is carried out upon CID-$MS^n$ measurement to such an extent that a parent ion does not disappear. Then, the remaining parent ion derived based on the theoretical plate with a number "n + 1" is further subjected to collision-induced dissociation (CID-$MS^{n+1}$ measurement) such that a purity test of a sugar chain structural isomer can be carried out. In a case in which a test sample is a pure product, consistency is observed for mass spectra derived from the CID-$MS^n$ and CID-$MS^{n+1}$ stage numbers and energy-dependent curves. In a case in which a test sample is a mixture comprising substances having identical mass numbers (structural isomer mixture), the composition ratio of a parent ion derived based on the CID-$MS^n$ stage number and that derived based on the CID-$MS^{n+1}$ stage number differ from each other so that the related obtained mass spectrometry results, particularly signal intensities or energy-dependent curves, differ from each other. The outline of the above purity test is shown in fig. 5.

Examples

The present invention is hereafter described in greater detail with reference to the following examples, although the technical scope of the present invention is not limited thereto.

Example 1 Confirmation of quantitativity of CID-$MS^n$ measurement

(1) Quantitativity of CID-MS/MS measurement values

When sugar chains comprising stereoisomers or bond positional isomers are subjected to CID-MS/MS measurement, the obtained CID energy-dependent curves are compared with each other, and the chemical structures of such sugar chains are determined, quantitativity of the obtained data must be considered. Thus, such quantitativity was confirmed. Fucα(1-3)Galβ-Octyl (which was synthesized by a method of PCT/JP04/009523) was dissolved in a solution of $H_2O$/methanol (1:1) to a final concentration of 10 ng/μl. The solution was loaded into a needle used for a CID-$MS^n$ measurement apparatus (esquire 3000 plus: Bruker Daltonics Corp.) by an infusion method (flow rate: 2 μl/min) with the use of a syringe pump, followed by measurement in a positive mode at a thermocapillary temperature of 250˚C, a capillary voltage of 4.0 kV, and a CID energy of 0.5 to 1.5 V.

Fig. 6A shows an ion chromatogram (TIC) of a fragment ion (m/z = 315) as an example of an ion graph that was actually obtained by the aforementioned measurement. In fig. 6A, a stepwise curve indicates voltage variation. The vertical axis and the horizontal axis stand for the total ion count and the measurement time, respectively.

Fig. 6B shows a graph (of CID energy-dependent curves) representing the relationship between the CID energy (voltage) and the total ion count in the cases of the ion (m/z = 315) used for the aforementioned measurement and its parent ion (m/z = 416). In fig. 6B, a CID energy-dependent curve indicated by circular plots is derived from the ion (m/z = 315) and a CID energy-dependent curve indicated by square plots is derived from the parent ion (m/z = 416). In both cases, the average of plots obtained by subtracting 2 plots immediately before voltage variation and 2 plots immediately after voltage variation from 16 plots was plotted.

In fig. 6B, the vertical axis represents the proportion of the total ion count in the case of m/z = 315 to the sum of the total ion count in the case of m/z = 315 and the total ion count of the parent ion (m/z = 416). With the use of such proportion, it becomes possible to compare a value with reference data without the influence of the ion counts of weak ions each having a different m/z.

In order to verify quantitativity of this graph, CV values (coefficients of variation) of ion counts at the following 3 points were calculated using 12 plots obtained by subtracting 2 plots of each upper limit from the above 16 plots: a point at which the leading edge of the CID energy-dependent curve in the case of m/z = 315 was observed (0.94 V); a point at which the CID energy-dependent curve crossed with an attenuating CID energy-dependent curve of a parent ion (m/z = 416) (1.12 V); and a point at which the CID energy-dependent curve reached a plateau (1.38 V). The results are shown in fig. 6C. The calculation equation is as follows: standard deviation/average x 100. Accordingly, CV values (coefficients of variation) at the individual voltages are 24.2%, 3.5%, and 7.3% in the cases of a point at which the leading edge of the CID energy-dependent curve was observed, a point at which the CID energy-dependent curve crossed with an attenuating CID energy-dependent curve of the parent ion, and a point at which the CID energy-dependent curve reached a plateau, respectively. In particular, the data obtained beyond a point at which the CID energy-dependent curve crossed with a CID energy-dependent curve of parent ion was highly reliable. Thus, it was shown that quantitative analysis can be carried out by the above method.

(2) Consistency of CID energy-dependent curves obtained via CID-$MS^n$ measurement

According to the present invention, regarding identical fragment ions, if CID energy-dependent curves obtained upon CID-MS/MS measurement differ from those obtained upon CID-MS/MS/MS measurement or CID-$MS^n$ measurement (n > 3), the obtained data cannot be compared with reference data. Thus consistency of CID energy-dependent curves was confirmed.

A pyridylaminated product (fig. 7A) of maltohexaose (hexasaccharide) was dissolved in a solution of $H_2O$/methanol (1:1) to a final concentration of 10 ng/μl. The resulting solution was loaded into a needle used for a CID-$MS^n$ measurement apparatus (esquire 3000 plus: Bruker Daltonics Corp.) by an infusion method (flow rate: 2 μl/min) using a syringe pump. Then, measurement was carried out in a positive mode at a thermocapillary temperature of 250°C, a capillary voltage of 4.0 kV, and a CID energy of 0.9 to 1 V. Fig. 7A-1 shows a CID energy-dependent curve in the case of m/z = 443 (disaccharide) obtained upon the above CID-MS/MS measurement. Herein, the vertical axis represents the proportion of the total ion count in the case of m/z = 443 to the sum of the total ion count in the case of m/z = 443 and the total ion count of a parent ion (m/z = 1091).

Next, a pyridylaminated product (fig. 7B) of maltoheptaose (heptasaccharide) was subjected to CID-MS/MS measurement in a manner similar to that used above. The obtained fragment ion (m/z = 1091) was further subjected to CID-MS (MS/MS/MS at this stage) measurement at a CID energy of 1.1 to 1.9 V. The thus obtained CID energy-dependent curve in the case of m/z = 443 (disaccharide) is shown in fig. 7B-1. Further, fig. 7C shows that the two above CID energy-dependent curves overlap each other. As is apparent from fig. 7C, CID energy-dependent curves of identical fragment ions correspond to each other even when such fragment ions were derived based on different stage numbers of CID-$MS^n$ measurement. Thus, the quantitativity of CID-$MS^n$ measurement was confirmed.

Example 2 Structure determination based on comparison of CID energy-dependent curves

(1) Consistency of CID energy-dependent curves derived from a naturally occurring sugar chain and a synthetic oligosaccharide chain

The present invention relates to a method of analyzing the structure of a target sugar chain, comprising comparing a CID energy-dependent curve of a reference sugar chain (library) and a CID energy-dependent curve of a naturally occurring sugar chain of unknown structure. In this Example, a synthetic sugar chain and a naturally occurring sugar chain that share the same structure and have different substituents were subjected to CID-MS/MS measurement such that parent ions having the same m/z were obtained. Then, the parent ions were further subjected to CID-MS/MS measurement. Thus, CID energy-dependent curves derived from the obtained daughter ions were found to be consistent with each other.

Fucα(1-6)Galβ(1-4)Glcβ-Octyl (synthetic product: fig. 8B) and $H_2O$-methanol (1:1) containing Fucα(1-2)Galβ(1-4)Glc (Dextra Lanoratories, Ltd.: fig. 8A) were subjected to CID-MS/MS measurement in a manner similar to that used in Example 1 (1). The obtained fragment ions (m/z = 305.08; cleaved glucose ring ($^{0,2}$A ion)) were further subjected to CID-MS/MS measurement. Fig. 8 shows CID energy-dependent curves in the case of m/z = 245 upon measurement. Fig. 8A-1 shows the curves derived from the naturally occurring sugar chain. Fig. 8B-1 shows the curves derived from the synthetic oligosaccharide having octyl. In addition, fig. 8C shows these curves overlap each other. As is apparent from fig. 8C, it was confirmed that identical CID energy-dependent curves can be obtained from fragment ions having the same m/z in the case of a combination of a naturally occurring sugar chain and a synthetic sugar chain and also in the case of a combination of a synthetic oligosaccharide and a naturally occurring sugar chain, even if parent ions derived based on the preceding stage number of CID-MS/MS measurement have different substituents.

In addition, the above CID energy-dependent curves were parameterized (with the use of Prism 4 (GraphPad Software)) by equation 1 with the use of indices such as the fiducial value (Bottom = 0) and the maximum value (Top = 100) serving as relative values of the ion count, CID energy ($V_{50}$) corresponding to 50% of the difference between the values, and the slope (Slope) at the inflection point of a CID energy-dependent curve, followed by comparison. As a result, almost identical values were obtained. Thus, it was confirmed that a partial structure of a naturally occurring sugar chain of unknown structure can be revealed by comparing parameters as described above.

(2) Consistency of CID energy-dependent curves derived from identical fragment ions obtained from sugar chains having different structures

The present invention relates to a method of analyzing the structure of a sugar chain, comprising performing CID-$MS^n$ measurement of a target sugar chain (having a structure differing from that of a parent fragment ion contained in reference data) until a fragment ion having a structure identical to that of the parent ion contained in reference data can be obtained, further performing CID-MS/MS measurement of the fragment ion, and comparing the thus obtained CID energy-dependent curve with that of reference data. Thus, two types of sugar chains having different structures were subjected to CID-$MS^n$ measurement until identical fragment ions were obtained. Then, the obtained fragment ions were further subjected to CID-MS/MS measurement. Accordingly, CID energy-dependent curves derived from the thus obtained daughter ions were found to be consistent with each other.

Sodium adduct ions of pyridylaminated (PA-) LeX pentasaccharide (fig. 9A) and LeX pentasaccharide (fig. 9B) having a free reducing end were subjected to CID-MS/MS measurement in the same manner as in Example 1. A fragment ion (m/z = 552.19) derived from a LeX-osyl residue (containing an LeX structure and an oxygen atom at the 1-position ofN-acetylglucosamine) was observed in both cases.

Thus, the fragment ion was further subjected to CID-MS/MS measurement (stage number of 3). Figs. 9C and 9D show the results of creating CID energy-dependent curves of fragment ions (m/z = 406 (9C) and 388 (9D)) based on the obtained plots. In the graphs of figs. 9C and 9D, circular plots are derived from pyridylaminated (PA-) LeX pentasaccharide and square plots are derived from LeX pentasaccharide having a free reducing end. As is apparent from figs. 9C and 9D, it was confirmed that CID energy-dependent curves derived from daughter ions having the same m/z (such daughter ions being obtained from fragment ions having identical structures) are consistent with each other even when such fragment ions are obtained from sugar chains having different structures.

(3) Identification of anomers

It was confirmed that it is possible to identify stereoisomeric fragment ions having identical structures by the method of the present invention. 8 types of anomers of Fuc$\alpha/\beta$(1-6)Gal$\alpha/\beta$(1-6)Glc$\alpha/\beta$-Octyl (fig. 10A), which differ from one another in terms of 3 glycosidic bonds, were chemically synthesized, followed by separation and purification by HPLC. Then, the resultants were subjected to CID-MS/MS measurement in the same manner as in Example 1. Figs. 10B and 10C each show an example of spectra upon the above MS/MS measurement of Fuc$\alpha$(1-6)Gal$\alpha$(1-6)Glc$\beta$-Octyl at different CID energies (10B: 0.82 V; 10C: 0.96 V). As is apparent from figs. 10B and 10C, a fragment ion (m/z = 477.2) was generated from a parent ion (m/z = 623.3) at a CID energy of 0.82 V. Further, two fragment ions (m/z = 477.2 and 331.1) were generated from the parent ion at 0.96 V.

Given that a sugar chain of unknown structure was used in the above analysis, it is understood that such sugar chain contains deoxyhexose and hexose based on the above results. However, it is unclear which glycosidic bond is contained in such sugar chain, such bond being derived from one of the above 8 types of anomers.

Thus, the fragment ion (m/z = 477.2) obtained above was further subjected to CID-MS/MS measurement (stage number of 3). The measurement conditions were the same as those used in Example 1. The measurement was carried out at a CID energy of 0.5 to 1.75 V. The fragment ion (m/z = 477.23) was found to be derived from 4 types of anomers of disaccharide from which monosaccharide (fucose) at the reducing end had been removed.

Fig. 10D shows the results of creating CID energy-dependent curves of fragment ions (m/z = 315) based on the obtained plots. In the graph of fig. 10, circular, square, triangular, and rhomboid plots represent glycosidic bonds $\alpha/\alpha$, $\beta/\alpha$, $\alpha/\beta$, and $\beta/\beta$, respectively. Also, in the graph, black plots represent a glycosidic bond with fucose that was released via CID (stage number of 1), referred to as "$\alpha$," and white plots represent such glycosidic bond, referred to as "$\beta$."

As is apparent from fig. 10D, CID energy-dependent curves derived from anomers obviously differed from one another. Thus, it was revealed that anomers can be identified by the analysis of the present invention. In addition, it was found that differences in anomers of parent ions do not influence CID energy-dependent curves of daughter ions.

Moreover, the above CID energy-dependent curves were parameterized (with the use of Prism 4 (GraphPad Software)) by equation 1 with the use of indices such as the fiducial value (Bottom = 0) and the maximum value (Top = 100) serving as relative values of the ion count, a CID energy ($V_{50}$) corresponding to 50% of the difference between the relative values, and the slope (Slope) at the inflection point of a CID energy-dependent curve, followed by comparison. Accordingly, different values were obtained from anomers. In addition, differences in anomers of parent ions do not influence the values of daughter ions. Thus, it was found that anomers also can be identified by the above calculation equation.

Accordingly, when a sugar chain of unknown structure is subjected to CID-$MS^n$ measurement, the obtained CID energy-dependent curves are compared with those of reference data, and some of the curves are found to be consistent with each other, a target sugar chain has a structure identical to that of the sugar chain from which the CID energy-dependent curves have been derived. Therefore, differences in anomers can be specified.

On the other hand, in a case in which CID energy-dependent curves obtained by performing CID-$MS^n$ measurement of a target sugar chain are not consistent with those of reference data related to a sugar chain having a structure identical to that of the target sugar chain, such sugar chain from which reference data is derived may be an anomer of the target sugar chain.

(4) Comparison of CID energy-dependent curves derived from different stage numbers of CID_$MS^n$ measurement

In accordance with the present invention, when CID energy-dependent curves of daughter ions derived from a fragment ion having a specific m/z value of a target sugar chain are consistent with those of reference data, there might be structural differences even though such consistency was observed in terms of the stage number of the measurement. Thus, when CID energy-dependent curves of other daughter ions obtained upon the same CID-$MS^n$ measurement were compared with CID energy-dependent curves of daughter ions having the same m/z of reference data, it was possible to perform structural analysis with improved accuracy. An example thereof is described below.

Two types of naturally occurring sugar chains each having a blood group O antigen epitope (Fucα(1-2)Gal) on the nonreducing end were selected as targets for comparison. These sugar chains shared the epitope (Fucα(1-2) Gal); however, their disaccharide structures containing galactose bound to fucose were a lacto series (fig. 11A) and a neolacto series (fig. 11B). Thus, they were bond positional isomers. Further, the sugar chain A was trisaccharide and the sugar chain B was pentasaccharide. Thus, they had obviously different mass numbers as parent ions. However, when sugar chains A and B were separately subjected to CID-$MS^n$ measurement, it was possible to obtain fragment ions sharing the same m/z (= 534.18) as sodium adducts, such ions being considered to be cleaved at a glucosaminyl bond (shown in fig. 11A and the left side of the line in fig. 11B).

Thus, these fragment ions were further subjected to CID-$MS^n$ measurement such that CID energy-dependent curves derived from their daughter ions (m/z = 388) were created. The curves were consistent with each other (fig. 11C). However, when CID energy-dependent curves derived from other daughter ions were created in a similar manner, it was revealed that such CID energy-dependent curves obviously differed from each other as shown in figs. 11D and 11E (daughter ions: m/z = 349 and m/z = 331, respectively). Accordingly, the structures of the above two types of sugar chains were found to differ from each other.

Example 3 Confirmation of possibility of identification based on structure isomerism via parameter analysis

First, it was examined whether or not it would be possible to determine anomer isomers and bond positional isomers with the use of oligosaccharides consisting of identical monosaccharides binding to each other via specific bonds. Maltooligosaccharide has a structure consisting of α(1-4) bonds of glucose, and cellooligosaccharide has a structure consisting of β(1-4) bonds of glucose, so that they are appropriate for use for anomer isomer comparison. In addition, isomaltooligosaccharide comprises an α(1-6) bond and thus it plays an important role for determination of bond position isomerism upon comparison with maltooligosaccharide.

(1) Determination of anomer isomerism

It was shown in Example 1(2) that there is a good correspondence between CID energy-dependent curves of maltooligosaccharide obtained upon CID-MS/MS measurement and those obtained upon MS/MS/MS measurement (CID-$MS^n$ and CID-$MS^{n+1}$). Herein, the obtained energy-dependent curves were subjected to sigmoid curve fitting. 3D scatter diagrams of the resulting parameters (Top, Slope, and $V_{50}$) were created and the 2D developments thereof were then created. Also, plotting was carried out in a manner similar to that for cellooligosaccharide in which bond positions are the same as those of maltooligosaccharide but differs therefrom merely in terms of anomer configuration (fig. 12). As a result, it was found that identical parameters are obtained in a case in which a precursor ion derived based on a stage number of 2 and one derived based on a stage number of 3 are identical to each other upon CID-$MS^n$ measurement. In particular, $V_{50}$ is mainly within the range of 1.40 to 1.45 V. Meanwhile, in the case of cellooligosaccharide, the $V_{50}$ value was almost 1.5 V. The result indicates that it is possible to determine the stage of bond cleavage based on differences of anomer configuration with the use of parameters.

(2) Identification of bond position isomerism

Parameters are obtained based on a collision energy map for isomaltooligosaccharide comprising an α(1-6) bond for comparison with the results for maltose obtained in Example 3(1). Then a graph was created from the obtained parameters and those derived from maltose (fig. 13). As a result, it was shown that the $V_{50}$ value can be used as an index of bond position isomerism.

Example 4 Identification of reference data relating to a trisaccharide library that can be used for a method for structural prediction

(1) Synthesis of a trisaccharide library and CID-$MS^n$ measurement of the same

Trisaccharide Fucα/β(1-6)Galα/β(1-2/3/4/6)Glcα/β-Octyl made up of fucose (Fuc), galactose (Gal), and glucose (Glc), which were contained in a trisaccharide library, was synthesized such that anomer mixtures were obtained. Then, each trisaccharide was isolated by HPLC followed by CID-$MS^n$ measurement. With the use of structural information regarding the obtained library compounds, structural prediction of the compounds of unknown structures was performed. Basically, there were 32 ($2^3 \times 4$) types of compounds as information sources. However, 23 compounds that had been possible to isolate were used herein for library structure data. All of the samples were subjected to CID-MS/MS measurement (m/z = 623 $[M + Na]^+$) and CID-MS/MS/MS measurement (m/z = 477 $[M- Fuc + Na]^+$).

(2) Data relating to dissociation of fucose residues derived from a trisaccharide library upon CID-MS/MS measurement

Each sample was subjected to CID-MS/MS measurement by procedures of Example 1 such that data was obtained (fig. 14). Thereafter, curve fitting (Boltzmann sigmoid function) was carried out. Then, parameters (Top, Slope, and $V_{50}$) were calculated (fig. 15). An example of Fucα/β(1-6)Galα/β(1-6)Glcβ-Octyl among the 23 compounds of the trisaccharide library is shown in figs. 14 and 15.

A scatter diagram of the calculated parameters was created. It is obviously understood that plots are divided into two groups. Also, focusing on anomer isomers (Fucα: black circles; Fucβ: white circles), it is obvious that plots reflect glycosidic bond structures (anomer isomerism) through fragmentation during a CID process (fig. 16). In such case, it is understood that parameters $V_{50}$ and Slope can be indices for identification of anomer isomerism. Only one plot (Slope) derived from a β form had a small value. However, such value was an error due to data dispersion. Thus, it can be statistically recognized as an outlier.

(3) Data relating to dissociation of galactose residues of a trisaccharide library upon CID-MS/MS/MS measurement

There is a tendency of polarization of data in the case of Gal disassociation (m/z = 315) upon CID-MS/MS/MS measurement as with the case of plots concerning Fuc disassociation upon CID-MS/MS measurement (fig. 17). This results from the fact that $V_{50}$ exclusively reflects an anomer isomer.

Example 5 Method for anomer identification with the use of a CID-$MS^n$ scatter diagram of a trisaccharide library

(1) Plotting range based on distances between plots and averages

Based on a graph of sigmoid curves obtained by CID-$MS^n$ measurement, it is understood that convergence is observed in the cases of an α coordination and a β coordination (explained in the cases of fucose and galactose of this Example) within the respective ranges. Upon anomer analysis of an unknown sugar chain, it is possible to carry out anomer prediction by examining whether or not results fall within their respective ranges.

First, in order to recognize dispersion ranges, the averages of $V_{50}$ values and Slope values derived from Fuc (α coordination) and those derived from Fuc (β coordination) were obtained. Values between each plots are determined to be range (R). Specific examples of such values in the case of Fucα(1-6)Galα(1-3)Glcβ-Octyl are described below:

The average of $V_{50}$ of all values of $V_{50}$-α = 0.00597; and
The average of Slope of all values of Slope-α = 0.000229.

A distance was obtained from each of the above values. The range of Fucα is represented by a circle having a radius of such distance (fig. 18). Values obtained from a Fucα library are shown in table 1.

[Table 1]

Table 1. Numerical values of calculated distances

| | | $V_{50}$ distance ($\alpha$V50-$\alpha$Ave.) | SLOPE | SLOPE distance ($\alpha$SLOPE-$\alpha$Ave.) | Distance |
|---|---|---|---|---|---|
| 0.8759 | | 0.00597 | 0.02884 | 0.000229 | 0.5974 x $10^{-2}$ |
| 0.8684 | | -0.00153 | 0.0286 | -1.1E-05 | 0.1530 x $10^{-2}$ |
| 0.8949 | | 0.02497 | 0.02718 | 0.02718 | 2.5011 x $10^{-2}$ |
| 0.8749 | | 0.00497 | 0.02774 | -0.000871 | 0.5046 x $10^{-2}$ |
| 0.8533 | | -0.01663 | 0.02604 | -0.002571 | 1.6828 x $10^{-2}$ |
| 0.8559 | | -0.01403 | 0.02758 | -0.001031 | 1.4068 x $10^{-2}$ |
| 0.8694 | | -0.00053 | 0.02937 | 0.000759 | 0.09257 x $10^{-2}$ |
| 0.8708 | | 0.00087 | 0.03002 | 0.001409 | 0.1656 x $10^{-2}$ |
| 0.8763 | | 0.00637 | 0.03077 | 0.002159 | 0.6726 x $10^{-2}$ |
| 0.8595 | | -0.01043 | 0.02997 | 0.001359 | 1.0518 x $10^{-2}$ |
| | | | | Ave, | 0.882827 x $10^{-2}$ |
| | ($\beta V_{50}$-$\beta$Ave.) | | $V_{50}$ distance | SLOPE distance ($\beta$SLOPE-$\beta$Ave.) | Distance |
| 163 BAB | 0.9553 | 0.017046154 | 0.04419 | 0.003145385 | 1.7333921 x $10^{-2}$ |
| 163 BBA | 0.9393 | 0.001046154 | 0.03922 | -0.001824615 | 0.21032494 x $10^{-2}$ |
| 163 BAA | 0.949 | 0.010746154 | 0.02918 | -0.011864615 | 1.6007769 x $10^{-2}$ |
| 163 BBB | 0.9332 | -0.005053846 | 0.04186 | 0.000815385 | 0.51192004 x $10^{-2}$ |
| 166 BAA | 0.9478 | 0.009546154 | 0.04305 | 0.002005385 | 0.97545184 x $10^{-2}$ |
| 166 BBB | 0.9145 | -0.023753846 | 0.04094 | -0.000104615 | 2.37540764 x $10^{-2}$ |
| 166 BAB | 0.9402 | 0.001946154 | 0.04173 | 0.000685385 | 0.20633147 x $10^{-2}$ |
| 166 BBA | 0.9178 | -0.019553846 | 0.04136 | 0.000315385 | 1.95563892 x $10^{-2}$ |
| 164 BAA | 0.9551 | 0.016846154 | 0.04228 | 0.001235385 | 1.68913906 x $10^{-2}$ |
| 164 BBA | 0.9319 | -0.006353846 | 0.04117 | 0.000125385 | 0.63550831 x $10^{-2}$ |
| 164 BBB | 0.9389 | 0.000646154 | 0.04162 | 0.000575385 | 0.08652069 x $10^{-2}$ |
| 162 BBB | 0.9212 | -0.017053846 | 0.0421 | 0.001055385 | 1.70864712 x $10^{-2}$ |
| 162BAB | 0.9522 | 0.013946154 | 0.04488 | 0.003835385 | 1.44639341 x $10^{-2}$ |
| | | | | Ave, | 1.164265572 x $10^{-2}$ |

(3) Correction of influences of molecular weights upon structural analysis

In Example 2, an energy-dependent curve derived from a partial structure of a compound constituting a sugar chain library obtained based on CID analysis of such compound was compared with an energy-dependent curve derived from a partial structure of the unknown substance. Thus, it has been shown that it is possible to predict a partial structure of an unknown substance if such curves are completely consistent with each other. Such prediction can be realized by referring to a library data for an identical fragment obtained from a different structure. In such case, ions trapped have the same molecular weight so that complete consistency of partial structures can be realized. However, upon mass spectrometry, the molecular weight (molecular size) of a parent ion is an important factor that influences results. Also, in this study, differences in molecular weights resulted in different results concerning curves and parameters (e.g., $V_{50}$ and Slope). Therefore, it is necessary for sugar chain structural analysis to be based on consideration of the influences of molecular weights. Hereafter, a method of correcting results obtained is provided (fig. 20).

A graph was created by plotting $V_{50}$ and Slope of a CID energy-dependent curve derived from a fragment ion (m/z = 477) of a sugar chain library (Fucα/β(1-6)Galα/β(1-2/3/4/6)Glcα/β-Octyl) and those of a CID energy-dependent curve derived from a reference daughter ion of Lewis a, which has m/z that is identical to that of a target daughter ion of an unknown sugar chain (herein used as a correction sample) (fig. 21). As is apparent from the graph, the plots were significantly influenced by the difference in their molecular weights. Next, based on such difference, correction values of $V_{50}$ and Slope were obtained by the equations shown below.

$V_{50}$ correction value = $V_{50}$ average of de-Fucα library (m/z = 477) / $V_{50}$ of Lewis a
Slope correction value = Slope average of de-Fucα library (m/z = 477) / Slope of Lewis a

The correction values calculated above were multiplied by $V_{50}$ and Slope obtained from a CID energy-dependent curve of the target daughter ion of the unknown sugar chain. As shown in fig. 21, it was suggested that a plot obtained from the unknown sugar chain before correction be, for example, Fucα, because the unknown sugar chain significantly differed from Fucβ. With the use of such method alone, it is impossible to specify what deoxyhexose is. However, a sugar chain that was used herein as the unknown sugar chain was a tetrasaccharide structure isolated from a human. In addition, since deoxyhexose that exists in humans is fucose, it was possible to predict that the unknown test sample would have a Fucα structure. The sugar chain that was actually used as the unknown sugar chain was Lewis X having a Fucα structure. Thus, with the use of the satisfactory library data, it is possible to carry out structural prediction with good accuracy.

(4) Correction of data from different apparatuses

Differences in data obtained from different apparatuses are problematic upon structural analysis with the use of mass spectrometers. Such differences were corrected by the above method. First, Fucα(1-6)Galα(1-6)Glcβ-Octyl was used as a correction sample. The apparatuses used for comparison were produced by the same company. However, Bruker QITMS was used for correction of "individual differences." Based on comparison of energy-dependent curve data, differences were found on the X axis (Ampl and $V_{50}$) (fig. 22). Next, with the use of the sample as a correction sample, correction in the case of Fucβ(1-6)Galβ(1-6)Glca-Octyl was carried out in a manner similar to that described in the paragraph immediately above the previous paragraph. As a result, data differences were reduced, suggesting that it was possible to carry out correction. With the use of a larger number of test samples for correction, correction accuracy can be improved.

## Example 6 Test examining contamination of structural isomers of a sugar chain

(1) Preparation of test samples and mass spectrometry

The following 4 types of test samples were prepared and measured in accordance with the method of Example 1. Specifically, injection of test samples into a mass spectrometer was carried out by an infusion method at a flow rate of 120 μl/h. The ion source applied (electro-spray ionization method: ESI) was used at a rate of 1 μl/min to 1 ml/min. In addition, on-line or off-line nano ESI was used such that it was possible to carry out analysis using the nl/min scale. The mass spectrometer used as a detector was a Bruker Daltonics esquire 3000 plus (QIT-$MS^n$). The analysis conditions are described below: 1) thermocapillary temperature: 205°C; 2) capillary voltage: 4.0 kV; and 3) Dry Guas: 4.0 1/min. upon measurement (positive ion mode). Herein, the isolation width as a condition for CID-$MS^n$ measurement was 0.8 ms in the absence of isotopes.

| | |
|---|---|
| Pure product A | Gal$\alpha$(1-3)Gal$\alpha$-OMe (7 nmol) |
| Pure product B | Gal$\alpha$(1-3)Gal$\beta$-OMe (7 nmol) |
| Mixture C | Pure product A: Pure product B = 1:1 |
| Mixture D | Pure product A: Pure product B = 1:9 |

(2) Results of analyzing pure product A and pure product B

Fig. 23 shows fragment strengths at individual Ampl voltages upon CID-MS/MS measurement, those upon CID-MS/MS/MS measurement, and differences therebetween in the case of pure product A. There were no substantial differences between the results obtained via CID-MS/MS measurement and those obtained via CID-MS/MS/MS measurement. Similar results were obtained from pure product B (fig. 24).

(3) Results of analyzing mixture C and mixture D

Figs. 25 and 26 show fragment strengths at individual Ampl voltages upon CID-MS/MS measurement, those upon CID-MS/MS/MS measurement, and differences therebetween in the case of mixture C and the same in the case of mixture D, respectively. There were significant differences in the results obtained in both mixture cases compared with the pure product cases. Based on such differences, it was possible to determine whether a sample was a mixture or a non-mixture.

Industrial Applicability

The method of analyzing the structure of a sugar chain of the present invention is a method of determining the structure of a sugar chain of unknown structure, comprising performing CID_MS$^n$ measurement of such sugar chain and comparing the obtained CID energy-dependent curves with that contained in reference data that is obtained from a sugar chain of known structure. In accordance with the method, it is possible to determine or predict a partial or the whole structure of a naturally occurring sugar chain of unknown structure. Such sugar chain may have a different substituent and a stereoisomer thereof also may be used. In addition, the method of the present invention is very useful in terms of analysis of a minute amount of such a sugar chain. Also, the method of the present invention may be used not only for a basic technology for next-generation life science studies, such as structural analysis of sugar chains, but also for a technology that contributes to medical diagnosis, such as identification of sugar chain receptors that induce diseases accompanied by sugar chain abnormality and infectious diseases. Further, in accordance with the purity test method of the present invention, the necessity of a test sample can readily be determined prior to detailed data analysis of such sample.

Brief Description of the Drawings

Fig. 1 shows the outline of the method of the present invention.
Fig. 2 shows the outline of the method of the present invention.
Fig. 3 shows the outline of the method of the present invention.
Fig. 4 shows the outline of the method of the present invention.
Fig. 5 shows the outline of the method of the present invention.
Fig. 6 shows a graph of results of CID-MS$^n$ measurement of Fuc$\alpha$(1-3)Gal$\beta$-Octyl.
Fig. 7 shows structures of maltohexaose and maltoheptaose, graphs showing results of CID-MS$^n$ measurement thereof, and a graph representing results of comparing the results of such CID-MS$^n$ measurement.
Fig. 8 shows structures of a naturally occurring product and a synthetic product of Fuc$\beta$(1-6)Gal$\beta$(1-4)Glc, graphs showing results of such CID-MS$^n$ measurement thereof, and a graph representing results of comparing the results of CID-MS$^n$ measurement.
Fig. 9 shows structures of pyridylaminated (PA-) LeX pentasaccharide and LeX pentasaccharide having a free reducing end, graphs showing results of CID-MS$^n$ measurement thereof, and a graph representing results of comparing the results of such CID-MS$^n$ measurement.
Fig. 10 shows a structure of an anomer of Fuc$\alpha$(1-6)Gal$\beta$(1-6)Glc$\beta$-Octyl, graphs showing results of CID-MS$^n$ measurement thereof, and a graph representing results of comparing the results of CID-MS$^n$ measurement.
Fig. 11 shows structures of two types of naturally occurring sugar chains each having a blood group O antigen epitope (Fucα(1-2)Gal) at the nonreducing end thereof, graphs showing results of CID-$MS^n$ measurement thereof, and a graph representing results of comparing the results of such CID-$MS^n$ measurement.

Fig. 12 shows 2-dimensionally developed graphs representing results of plotting cleavage ion parameters (Top, Slope, and $V_{50}$) derived from a CID energy-dependent curve of maltohexaose and those derived from a CID energy-dependent curve of maltoheptaose.

Fig. 13 shows a 2-dimensionally developed graph representing results of plotting cleavage ion parameters (Top, Slope, and $V_{50}$) derived from a CID energy-dependent curve of maltohexaose and those derived from a CID energy-dependent curve of isomaltohexaose.

Fig. 14 shows a graph representing results of CID-$MS^n$ measurement of Fucα(1-6)Galα(1-6)Glcβ-Octyl that is an anomer of Fucα/β(1-6)Galα/β(1-2/3/4/6)Glcα/β-Octyl constituting a trisaccharide library.

Fig. 15 shows a graph representing results of curve fitting of a CID energy-dependent curve of Fucα(1-6)Galα(1-6) Glcβ-Octyl and values of cleavage ion parameters (Top, Slope, and $V_{50}$) that are obtained from the graph.

Fig. 16 shows a 2-dimensinally developed graph representing results of plotting cleavage ion parameters (Top, Slope, and $V_{50}$) resulting from dissociation of a fucose residue of each anomer of Fucα/β(1-6)Galα/β(1-2/3/4/6) Glcα/β-Octyl constituting a trisaccharide library. Each anomer is represented by the abbreviation of the bond positional isomerism. For instance, "163AAB" stands for Fucα(1-6)Galα(1-3)Glcβ-Octyl.

Fig. 17 shows a 2-dimensionally developed graph representing results of plotting cleavage ion parameters (Slope and $V_{50}$) resulting from dissociation of a galactose residue of each anomer of Fucα/β(1-6)Galα/β(1-2/3/4/6)Glcα/β-Octyl constituting a trisaccharide library.

Fig. 18 shows a graph representing the scope of possible prediction of an anomer of an unknown sugar chain based on a 2-dimentional development showing averages of cleavage ion parameters constituting a trisaccharide library.

Fig. 19 shows a 2-dimensionally developed graph representing results of plotting averages (at α coordination and β coordination) of cleavage ion parameters (Slope and $V_{50}$) resulting from dissociation of fucose of anomers constituting a trisaccharide library, the standard deviations of the averages, and cleavage ion parameters resulting from dissociation of fucose of Fucβ(1-6)Galα(1-6)Glcα-Octyl.

Fig. 20 shows the outline of a correction method in a case in which the molecular weight of a reference daughter ion differs from that of a target daughter ion.

Fig. 21 shows a 2-dimensionally developed graph of cleavage ion parameters (Slope and $V_{50}$) obtained in a case in which the molecular weight of a reference daughter ion differs from that of a target daughter ion, such graph representing the relationship between a measurement value and a correction value.

Fig. 22 shows a graph representing results of CID-$MS^n$ measurement of Fucα(1-6)Galα(1-6)Glcβ-Octyl with the use of different types of mass spectrometers.

Fig. 23 shows graphs showing results of CID-MS/MS measurement and CID-MS/MS/MS measurement of pure product A (Galα(1-3)Galα-OMe), which is a disaccharide, at amplitude voltages (Ampl V) at which the ratios of a parent ion to a fragment ion having the highest strength are approximately 1:3, 1:1, and 3:1, respectively.

Fig. 24 shows graphs showing results of CID-MS/MS measurement and CID-MS/MS/MS measurement of pure product B (Galα(1-3)Galβ-OMe), which is a disaccharide, at amplitude voltages (Ampl V) at which the ratios of a parent ion to a fragment ion having the highest strength are approximately 1:3, 1:1, and 3:1, respectively.

Fig. 25 shows graphs showing results of CID-MS/MS measurement and CID-MS/MS/MS measurement of a mixture C obtained by mixing pure product A (Galα(1-3)Galα-OMe) and pure product B (Galα(1-3)Galβ-OMe), each of which is a disaccharide, at a ratio of 1:1 at amplitude voltages (Ampl V) at which the ratios of a parent ion to a fragment ion having the highest strength are approximately 1:3, 1:1, and 3:1, respectively.

Fig. 26 shows graphs showing results of CID-MS/MS measurement and CID-MS/MS/MS measurement of a mixture C obtained by mixing pure product A (Galα(1-3)Galα-OMe) and pure product B (Galα(1-3)Galβ-OMe), each of which is a disaccharide, at a ratio of 1:9 at amplitude voltages (Ampl V) at which the ratios of a parent ion to a fragment ion having the highest strength are approximately 1:3, 1:1, and 3:1, respectively.

## Claims

**1.** A method of analyzing the structure of a sugar chain, comprising: (a) performing CID-$MS^n$ measurement of a target sugar chain until a fragment ion having a specific m/z is obtained; (b) further performing CID-MS/MS measurement of the fragment ion and creating a CID energy-dependent curve that shows the relationship between the CID energy and the total ion count of the obtained daughter ion having a specific m/z; and (c) comparing the CID energy-dependent curve with a CID energy-dependent curve of a daughter ion having an m/z identical to that of the above daughter ion, which is obtained via CID-MS/MS measurement of a fragment ion that has an m/z identical to that of the above fragment ion and is obtained from a reference sugar chain of known structure.

**2.** A CID energy-dependent curve library comprising CID energy-dependent curves showing the relationships between the CID energies and the total ion counts of a plurality of daughter ions each having a specific m/z, which are obtained via CID-$MS^n$ measurement of a plurality of reference sugar chains of known structures.

**3.** The library according to claim 2, wherein each reference sugar chain comprises trisaccharide.

**4.** A cleavage ion parameter library comprising cleavage ion parameters of a plurality of reference sugar chains of known structures, which is created from the CID energy-dependent curves according to claim 2.

**5.** The library according to claim 4, wherein the cleavage ion parameters of a plurality of reference sugar chains of known structures comprise at least one parameter selected from the group consisting of a relative value represented by the maximum value of ionic strength of a daughter ion having a specific m/z with respect to the total ion count of the daughter ion, a CID energy value at which 50% of the above relative value is obtained, and the slope at the inflection point of the CID energy-dependent curve.

**6.** The library according to claim 4 or 5, wherein each reference sugar chain comprises trisaccharide.

**7.** A method of analyzing the structure of a sugar chain, comprising: (a) performing CID-$MS^n$ measurement of a target sugar chain until a fragment ion having a specific m/z is obtained; (b) further performing CID-MS/MS measurement of the fragment ion and creating a CID energy-dependent curve showing the relationship between the CID energy and the total ion count of the obtained daughter ion having a specific m/z; and (c) comparing the CID energy-dependent curve with a CID energy-dependent curve of a daughter ion having an m/z identical to that of the above daughter ion, which is obtained by performing CID-MS/MS measurement of a fragment ion having an m/z identical to that of the above fragment ion contained in the library of claim 2 or 3.

**8.** The method according to claim 1 or 7, comprising creating cleavage ion parameters of a CID energy-dependent curve of a daughter ion having a specific m/z that is obtained from a target sugar chain in step (c) according to claim 1 or 7 and comparing the cleavage ion parameters with cleavage ion parameters of a CID energy-dependent curve of a daughter ion having an m/z identical to that of the above daughter ion of the reference sugar chain of known structure.

**9.** The method according to claim 8, wherein cleavage ion parameters comprise at least one parameter selected from the group consisting of a relative value represented by the maximum value of ionic strength of a daughter ion having a specific m/z that is relative to the total ion count of the daughter ion, a CID energy value at which 50% of the above relative value is obtained; and the slope at the inflection point of the CID energy-dependent curve.

**10.** A method of analyzing the structure of a sugar chain, comprising: (a) performing CID-$MS^n$ measurement of a target sugar chain until a fragment ion having a specific m/z is obtained; (b) further performing CID-MS/MS measurement of the fragment ion and creating a CID energy-dependent curve showing the relationship between the CID energy and the total ion count of the obtained daughter ion having a specific m/z; (c) creating cleavage ion parameters of the CID energy-dependent curve; (d) comparing the cleavage ion parameters with cleavage ion parameters created from a CID energy-dependent curve of a daughter ion having an m/z identical to that of the above daughter ion, which is obtained via CID-MS/MS measurement of a fragment ion having an m/z identical to that of the above fragment ion contained in a library according to claims 4 to 6.

**11.** The method according to claim 1 or any one of claims 7 to 10, wherein the total ion count of the daughter ion having a specific m/z, from which a CID energy-dependent curve is obtained, is represented by the proportion of the total ion count of the daughter ion having a specific m/z to the sum of the total ion count of such daughter ion and the total ion count of the parent ion.

**12.** A method of analyzing the structure of a sugar chain, comprising allowing fragment ions having different specific m/z levels contained in a target sugar chain to be repeatedly subjected to the steps (a) to (c) in claim 1 or 7 or the steps (a) to (d) in claim 10 and combining the obtained results.

**13.** A system for analyzing the structure of a sugar chain, comprising at least a CID-$MS^n$ measurement apparatus, a recording medium on which the library according to any one of claims 2 to 6 is recorded so as to be readable by a computer, and a recording medium on which a program used for allowing different specific fragment ions contained in a target sugar chain to be subjected to steps (b) and (c) in claim 1 or 7 or the steps (b) to (d) in claim 10 is recorded.

**14.** A method of examining contamination of a sugar chain structural isomer, comprising: (a) performing CID_$MS^n$ measurement of a sugar chain test sample at a given voltage at which a fragment ion having a specific m/z does not disappear; (b) further performing CID-MS/MS measurement of the remaining fragment ion; and (c) comparing mass spectra, CID energy-dependent curves, or cleavage ion parameters of such curves, which are obtained in the above two steps, with each other.

Fig.1

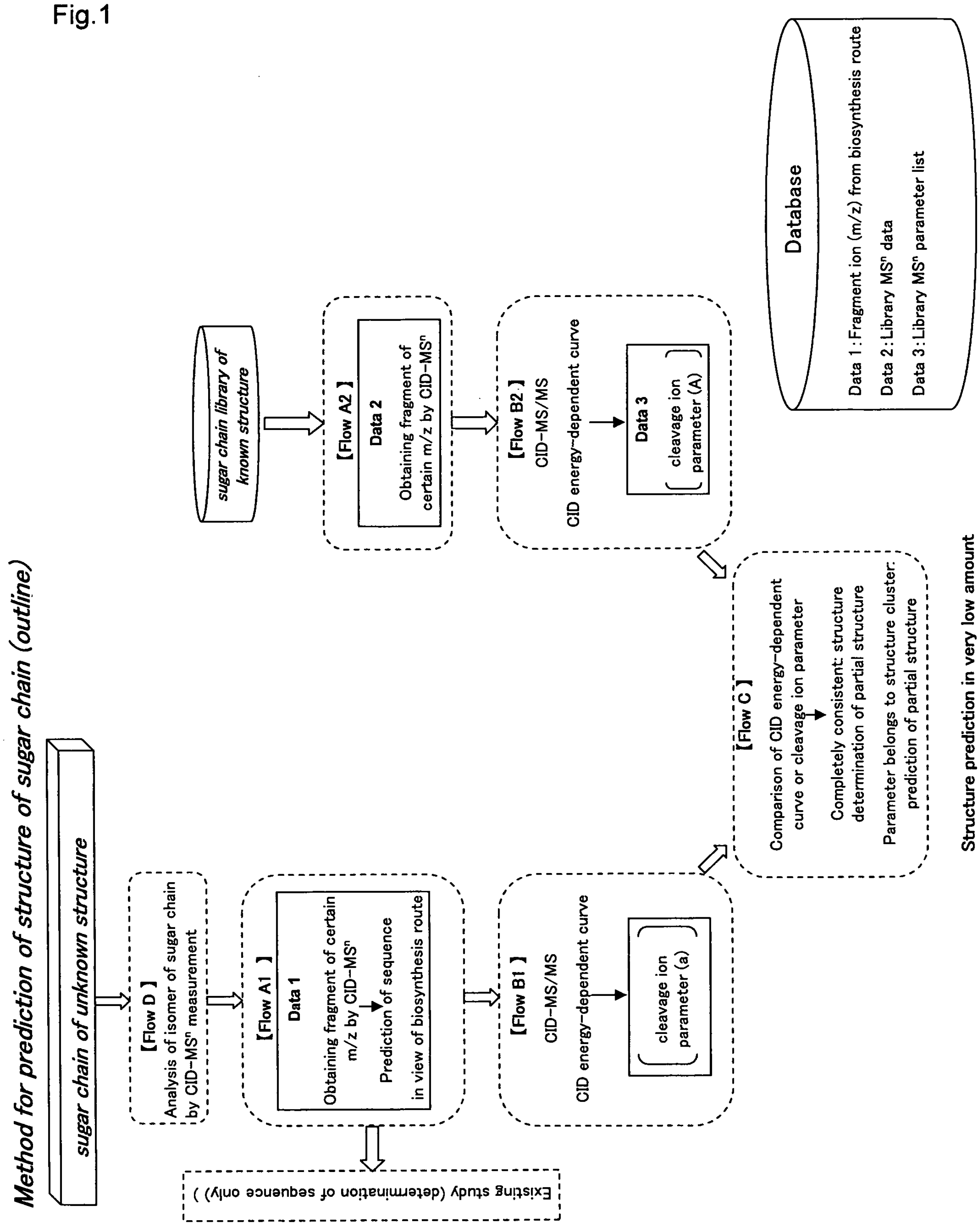
Method for prediction of structure of sugar chain (outline)
sugar chain of unknown structure
【Flow D】
Analysis of isomer of sugar chain by CID-MS$^n$ measurement
【Flow A1】
Data 1
Obtaining fragment of certain m/z by CID-MS$^n$
Prediction of sequence in view of biosynthesis route
Existing study (determination of sequence only)
【Flow B1】
CID-MS/MS
CID energy-dependent curve
cleavage ion parameter (a)
sugar chain library of known structure
【Flow A2】
Data 2
Obtaining fragment of certain m/z by CID-MS$^n$
【Flow B2】
CID-MS/MS
CID energy-dependent curve
Data 3
cleavage ion parameter (A)
【Flow C】
Comparison of CID energy-dependent curve or cleavage ion parameter
Completely consistent: structure determination of partial structure
Parameter belongs to structure cluster: prediction of partial structure
Structure prediction in very low amount
Database
Data 1: Fragment ion (m/z) from biosynthesis route
Data 2: Library MS$^n$ data
Data 3: Library MS$^n$ parameter list

Fig.2

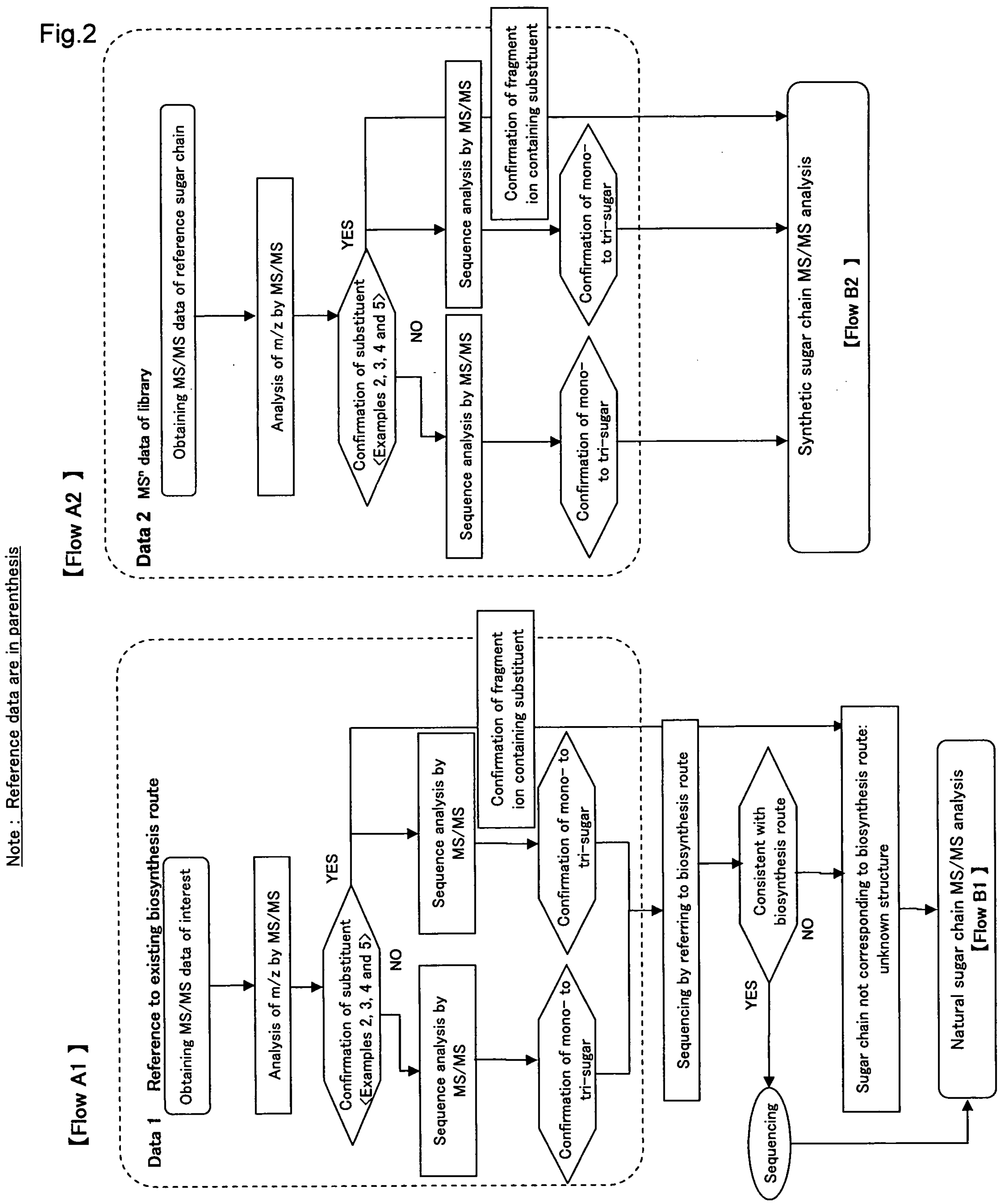
Note : Reference data are in parenthesis
【Flow A1 】
Data 1 Reference to existing biosynthesis route
Obtaining MS/MS data of interest
Analysis of m/z by MS/MS
Confirmation of substituent 〈Examples 2, 3, 4 and 5〉
YES
NO
Sequence analysis by MS/MS
Sequence analysis by MS/MS
Confirmation of fragment ion containing substituent
Confirmation of mono- to tri-sugar
Confirmation of mono- to tri-sugar
Sequencing by referring to biosynthesis route
Consistent with biosynthesis route
YES
NO
Sequencing
Sugar chain not corresponding to biosynthesis route: unknown structure
Natural sugar chain MS/MS analysis 【Flow B1 】
【Flow A2 】
Data 2 MS$^n$ data of library
Obtaining MS/MS data of reference sugar chain
Analysis of m/z by MS/MS
Confirmation of substituent 〈Examples 2, 3, 4 and 5〉
YES
NO
Sequence analysis by MS/MS
Sequence analysis by MS/MS
Confirmation of fragment ion containing substituent
Confirmation of mono- to tri-sugar
Confirmation of mono- to tri-sugar
Synthetic sugar chain MS/MS analysis 【Flow B2 】

Fig.3

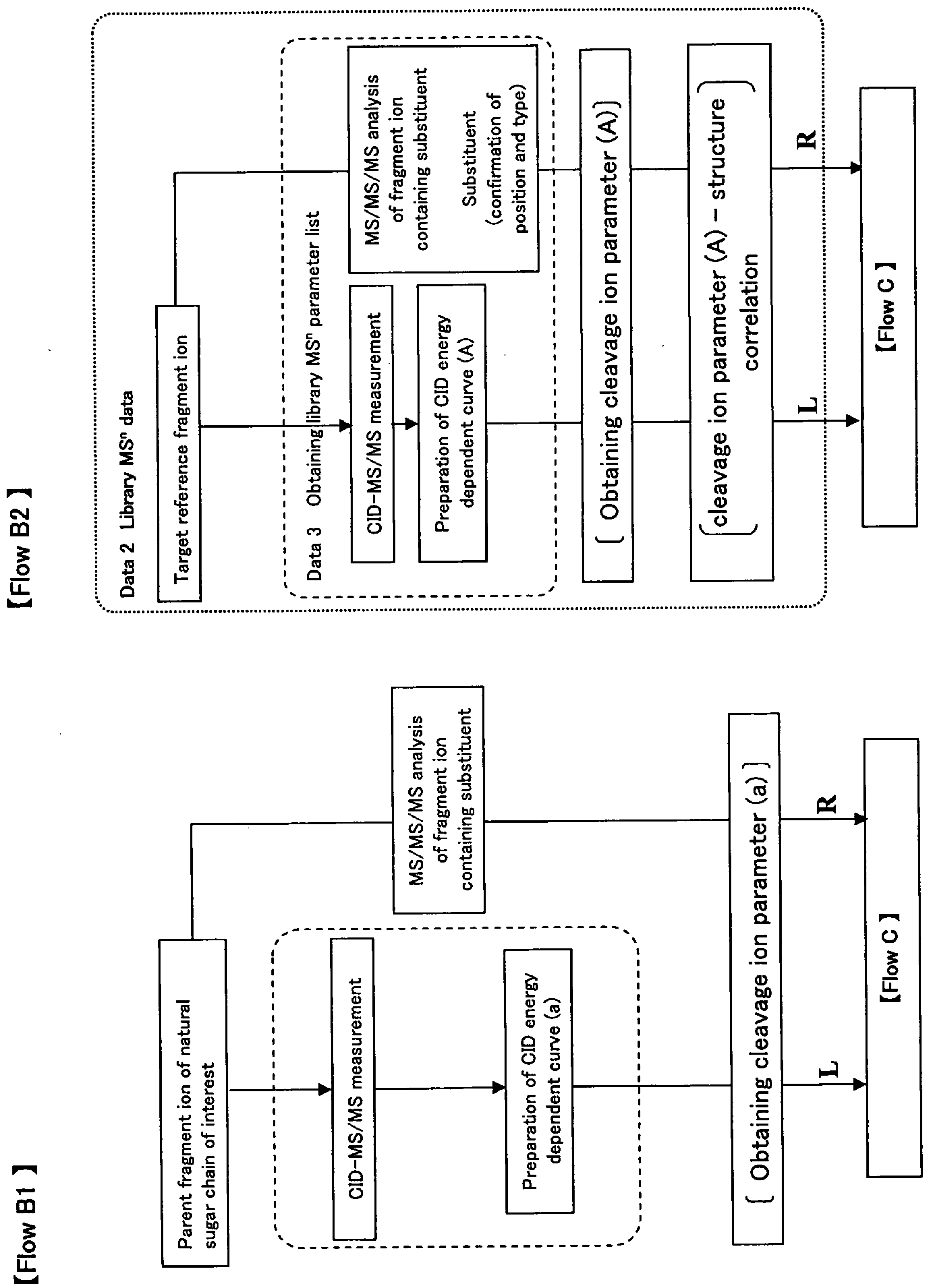
【Flow B2】
Data 2 Library MS^n data
Target reference fragment ion
Data 3 Obtaining library MS^n parameter list
CID-MS/MS measurement
Preparation of CID energy dependent curve (A)
MS/MS/MS analysis of fragment ion containing substituent
Substituent (confirmation of position and type)
〔Obtaining cleavage ion parameter (A)〕
〔cleavage ion parameter (A) – structure correlation〕
L
R
【Flow C】
【Flow B1】
Parent fragment ion of natural sugar chain of interest
CID-MS/MS measurement
Preparation of CID energy dependent curve (a)
MS/MS/MS analysis of fragment ion containing substituent
〔Obtaining cleavage ion parameter (a)〕
L
R
【Flow C】

Fig.4

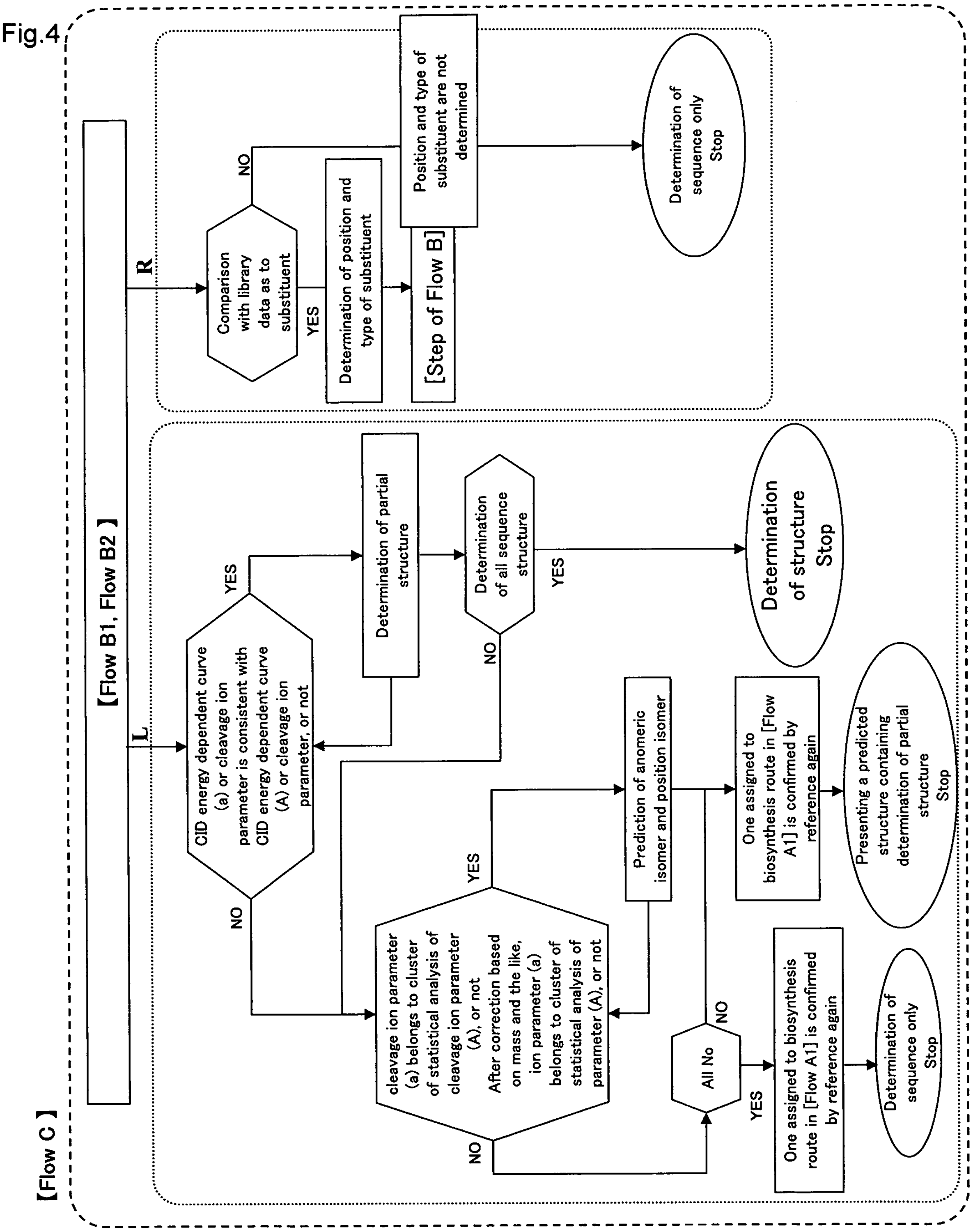
【Flow C】
【Flow B1, Flow B2】
R
L
Comparison with library data as to substituent
YES
NO
Determination of position and type of substituent
[Step of Flow B]
Position and type of substituent are not determined
Determination of sequence only Stop
CID energy dependent curve (a) or cleavage ion parameter is consistent with CID energy dependent curve (A) or cleavage ion parameter, or not
YES
NO
Determination of partial structure
Determination of all sequence structure
YES
NO
Determination of structure Stop
cleavage ion parameter (a) belongs to cluster of statistical analysis of cleavage ion parameter (A), or not After correction based on mass and the like, ion parameter (a) belongs to cluster of statistical analysis of parameter (A), or not
YES
NO
Prediction of anomeric isomer and position isomer
One assigned to biosynthesis route in [Flow A1] is confirmed by reference again
Presenting a predicted structure containing determination of partial structure Stop
All No
YES
NO
One assigned to biosynthesis route in [Flow A1] is confirmed by reference again
Determination of sequence only Stop

Fig.5

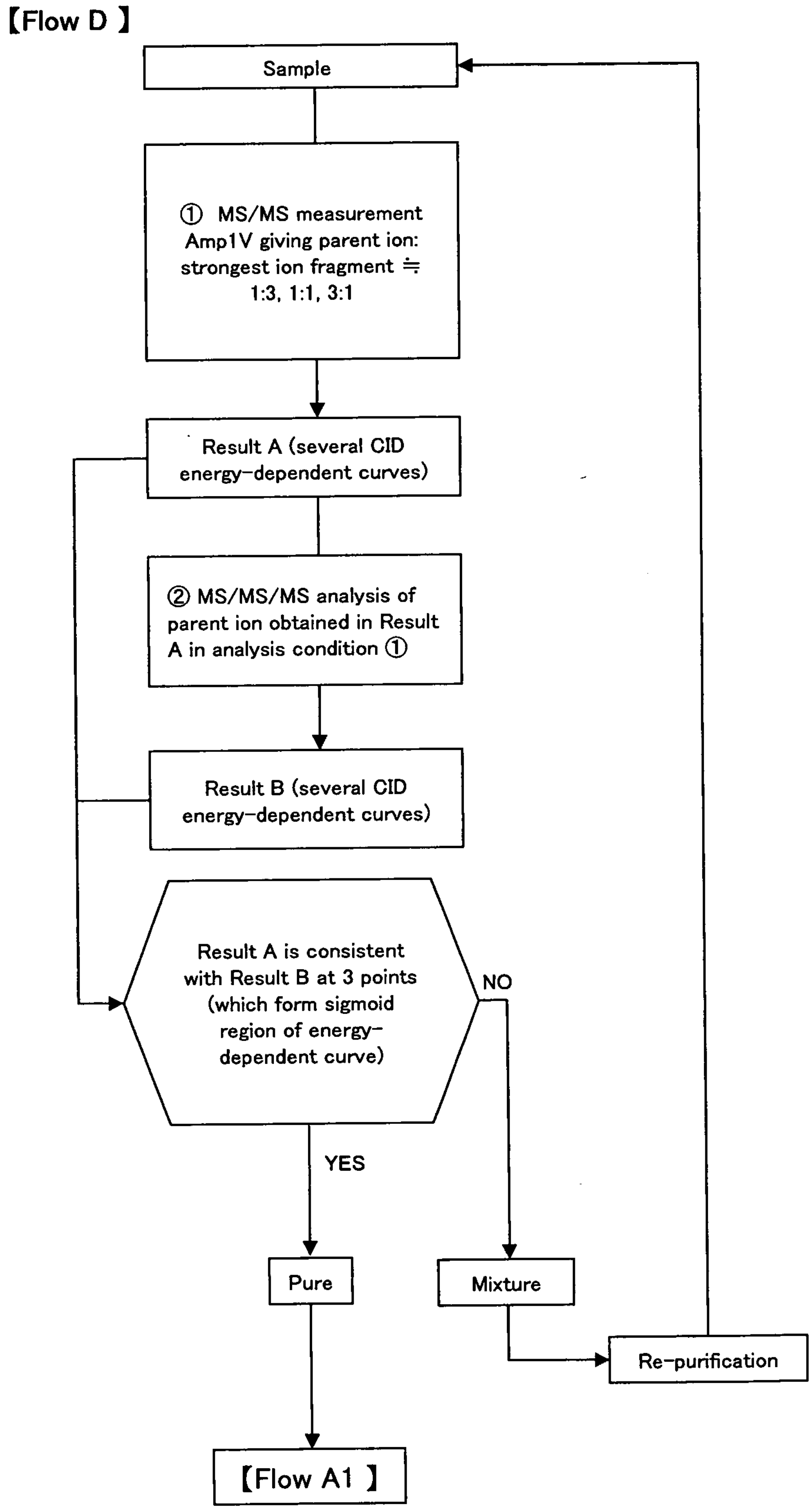
【Flow D】
Sample
① MS/MS measurement
Amp1V giving parent ion:
strongest ion fragment ≒
1:3, 1:1, 3:1
Result A (several CID
energy-dependent curves)
② MS/MS/MS analysis of
parent ion obtained in Result
A in analysis condition ①
Result B (several CID
energy-dependent curves)
Result A is consistent
with Result B at 3 points
(which form sigmoid
region of energy-
dependent curve)
NO
YES
Pure
Mixture
Re-purification
【Flow A1】

Fig. 6

A B

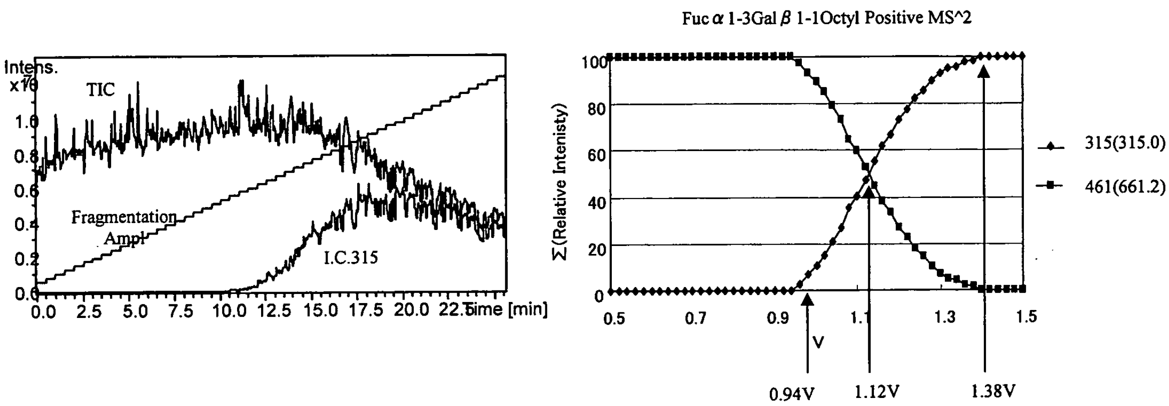


C

| CE(V) | 0.94(Starting) | 1.12(intersection ) | 1.38(plateau ) |
|---|---|---|---|
| | 85703 | 3674705 | 4848948 |
| | 98677 | 3538478 | 3954799 |
| | 134145 | 3686257 | 4717678 |
| | 164405 | 3556614 | 4597546 |
| | 162222 | 3924140 | 4698670 |
| | 152782 | 3734868 | 3931785 |
| | 194815 | 3641367 | 4767321 |
| | 181738 | 3873961 | 4922475 |
| | 216784 | 3670521 | 4832336 |
| | 167279 | 3641239 | 4901664 |
| | 196960 | 3782634 | 4739729 |
| | 158269 | 3907070 | 4786795 |
| Aev. | 159481.5833 | 3719321.167 | 4641645.5 |
| Standard deviation | 38557.30169 | 128693.0539 | 338302.265 |
| C.V.(%) | 24.17664842 | 3.460122106 | 7.288412374 |

Fig. 7

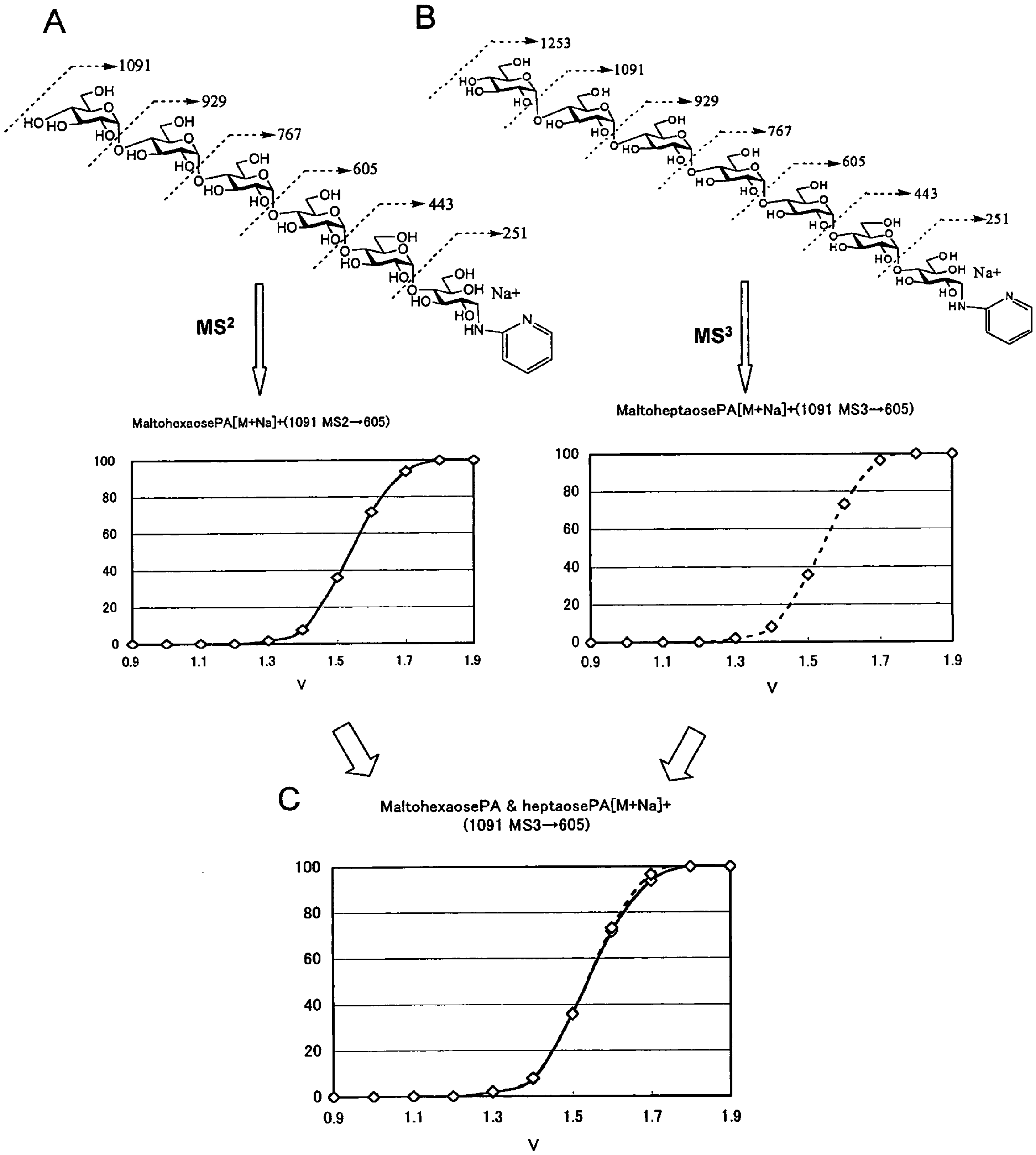
A
B
MS2
MS3
MaltohexaosePA[M+Na]+(1091 MS2→605)
MaltoheptaosePA[M+Na]+(1091 MS3→605)
C
MaltohexaosePA & heptaosePA[M+Na]+
(1091 MS3→605)
V

Fig. 8

A

**Fucβ 1-2Galβ 1-4Glc (Natural product)**

B

**Fucβ 1-6Galβ 1-4Glc1-1Octyl (Synthesized product)**

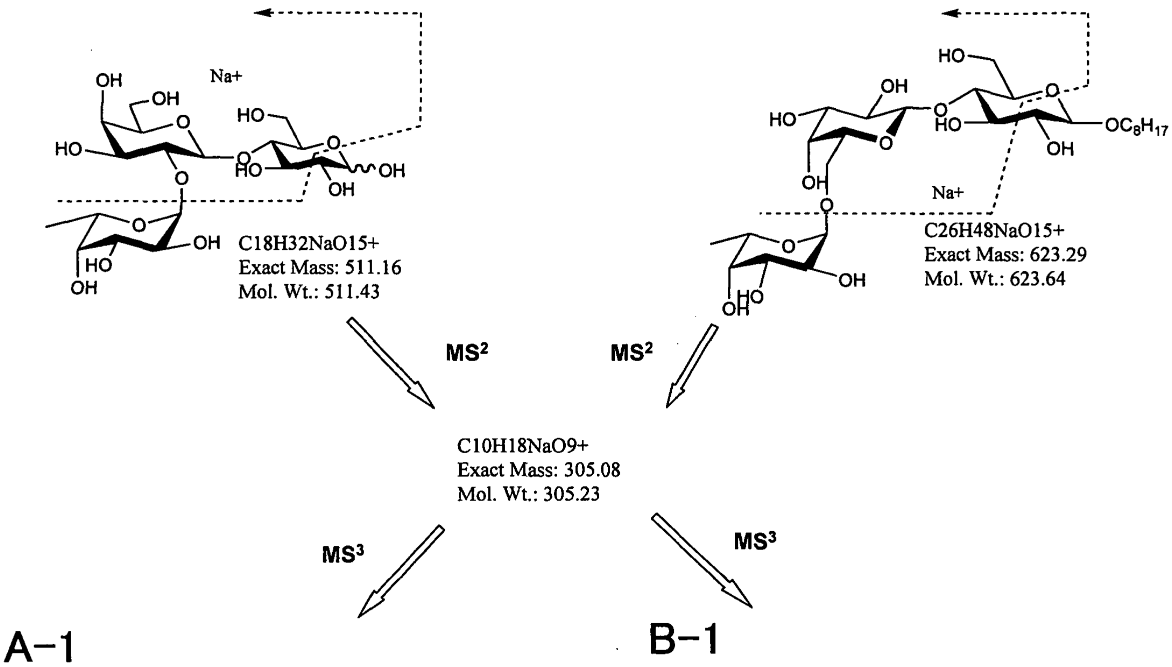


A-1

Fucβ 1-2Galβ 1-4Glc MS/MS/MS

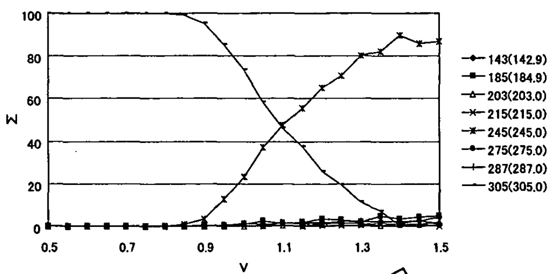


B-1

Fucβ 1-6Galβ 1-4Glcβ 1-1Octyl MS/MS/MS

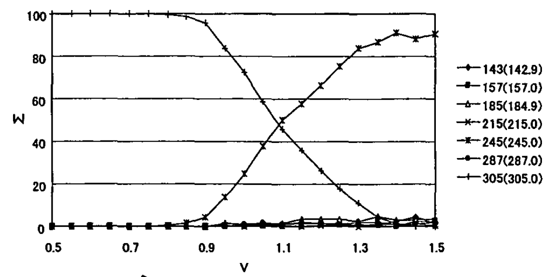


C

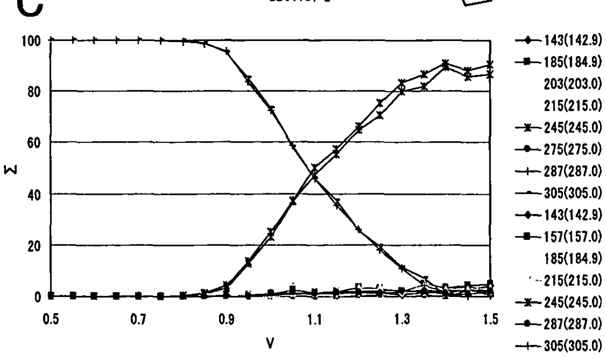

Fig.9

A

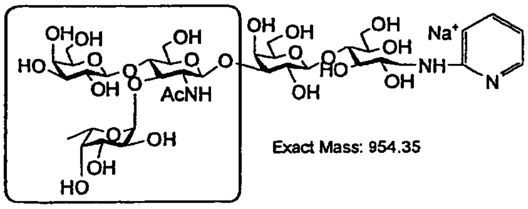

Exact Mass: 954.35

Lewis x 552.19

B

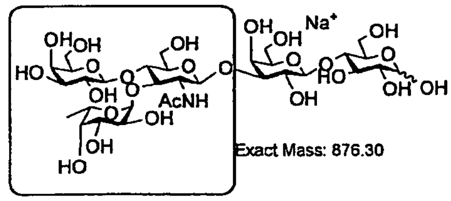

Exact Mass: 876.30

Lewis x 552.19

C 552→388 Generation

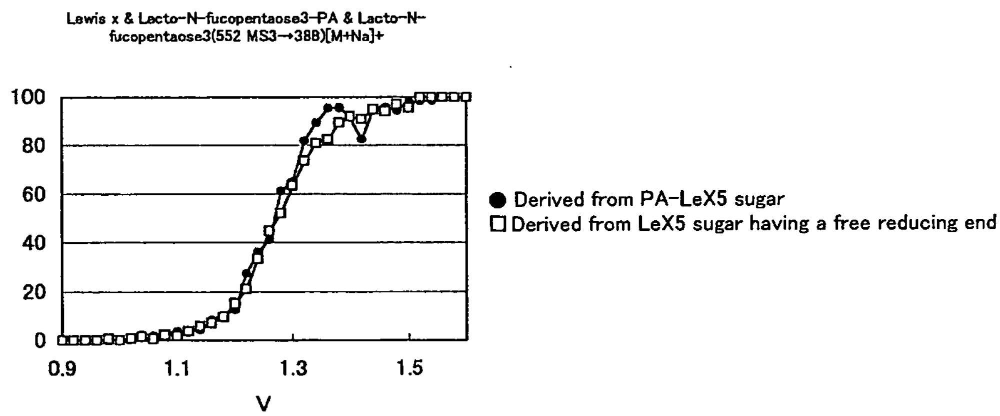


D 552→406 Generation

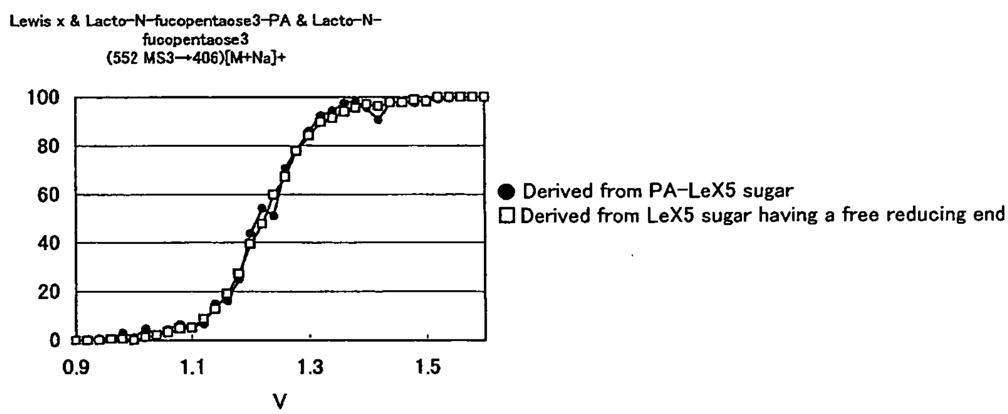

Fig.10

**A**

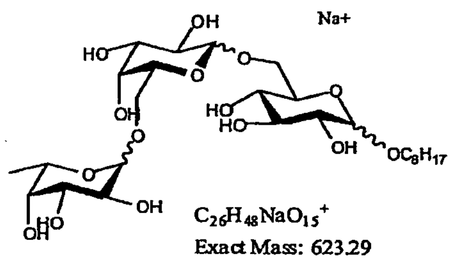

$C_{26}H_{48}NaO_{15}^+$
Exact Mass: 623.29
Mol. Wt.: 623.64

**B**

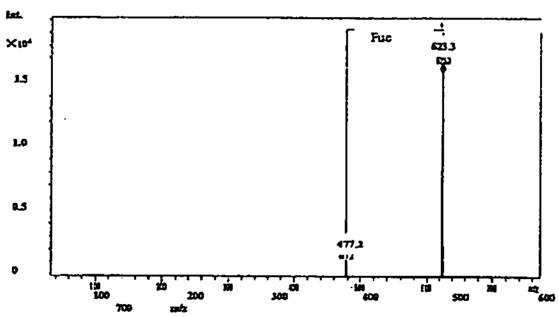


**C**

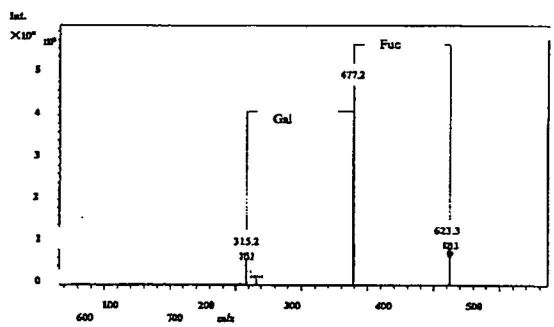


**D**

Fuc1-6Gal1-6Glc1-1Octyl

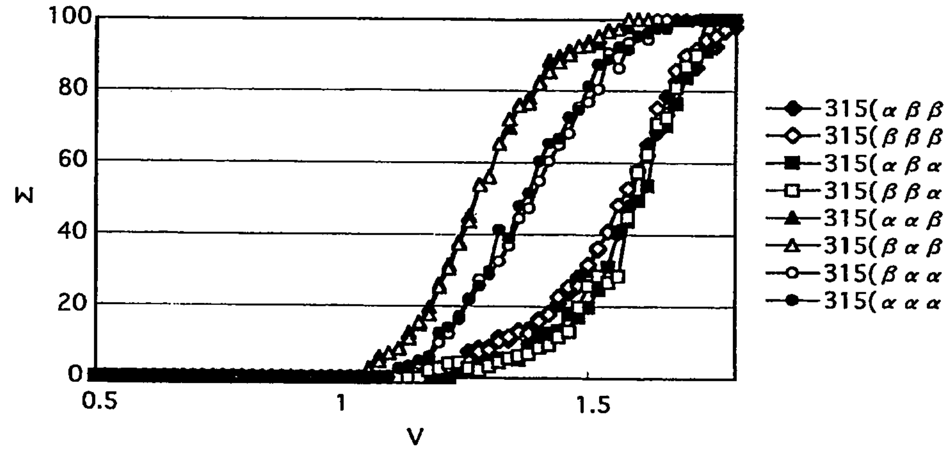

Fig.11

A

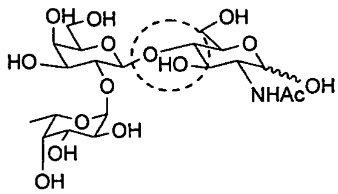

B

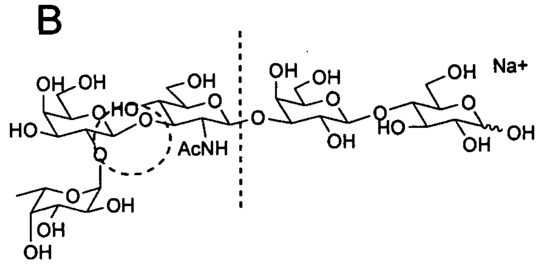

A-1

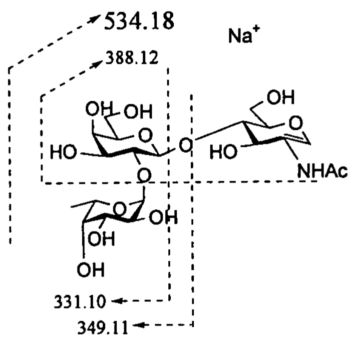


B-1

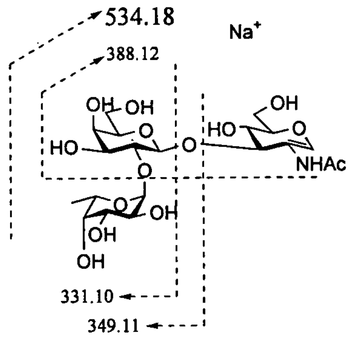


C 534 $MS^3$→388

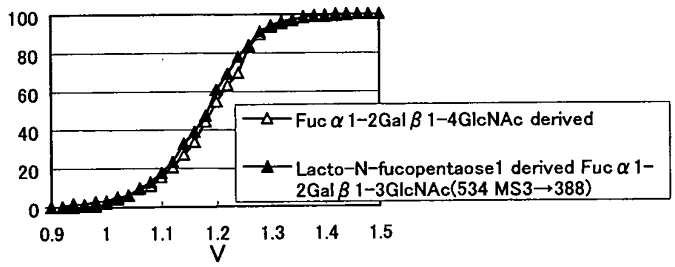


D 534 $MS^3$→349

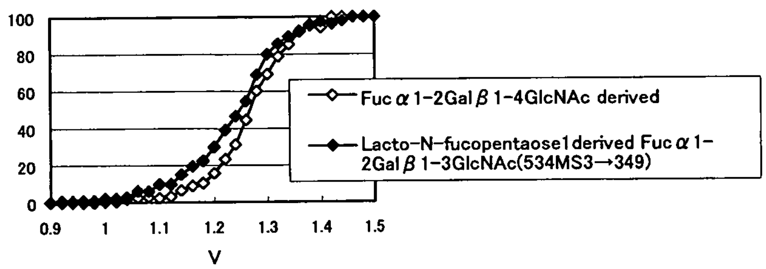


E 534 $MS^3$→331

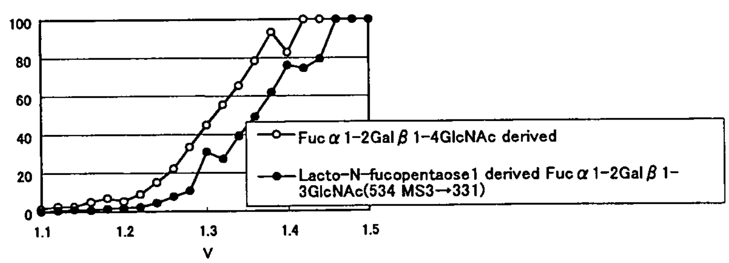

Fig.12

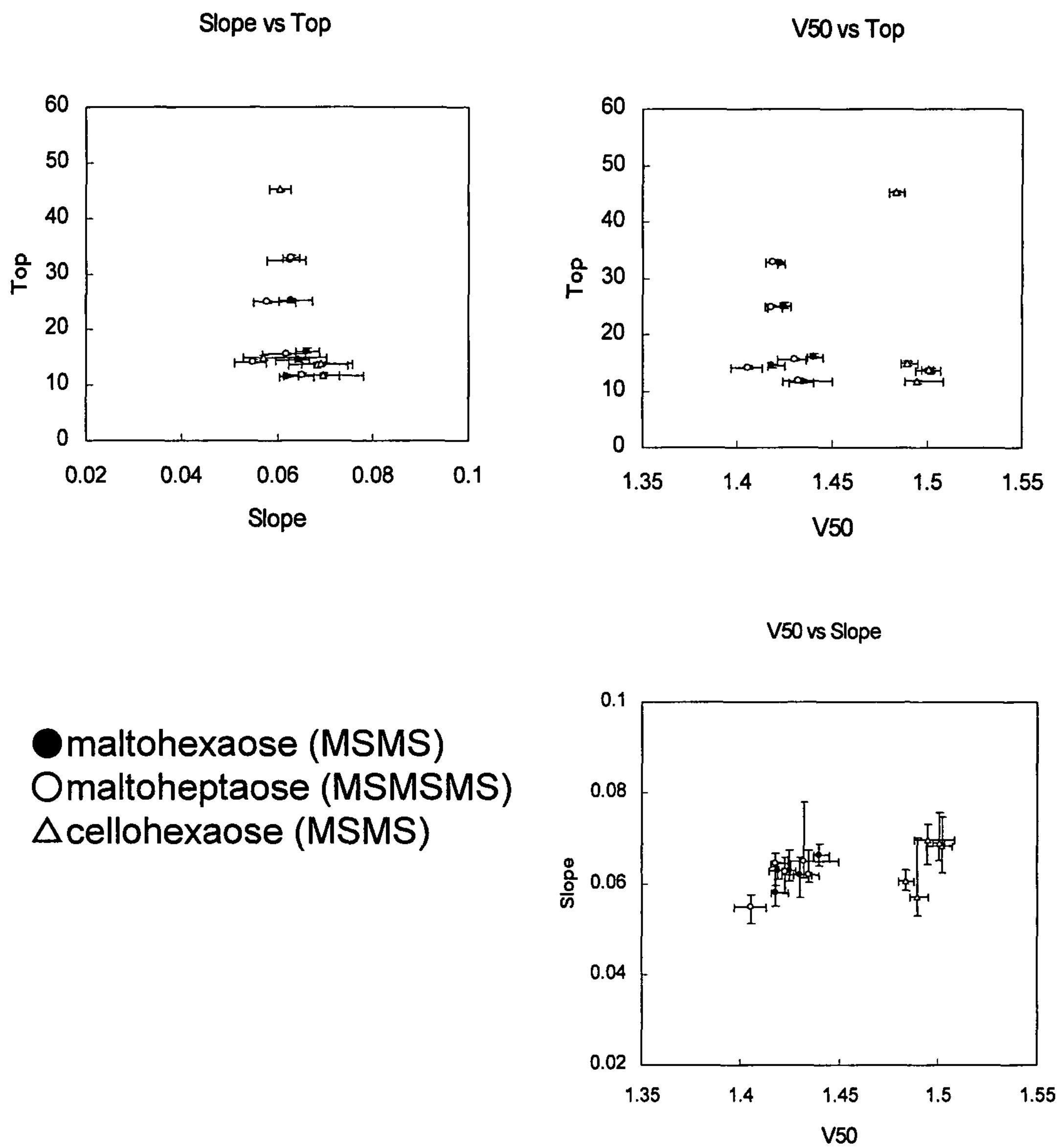
Slope vs Top
Top
Slope
0
10
20
30
40
50
60
0.02
0.04
0.06
0.08
0.1
V50 vs Top
Top
V50
0
10
20
30
40
50
60
1.35
1.4
1.45
1.5
1.55
V50 vs Slope
Slope
V50
0.02
0.04
0.06
0.08
0.1
1.35
1.4
1.45
1.5
1.55
●maltohexaose (MSMS)
○maltoheptaose (MSMSMS)
△cellohexaose (MSMS)

Fig.13

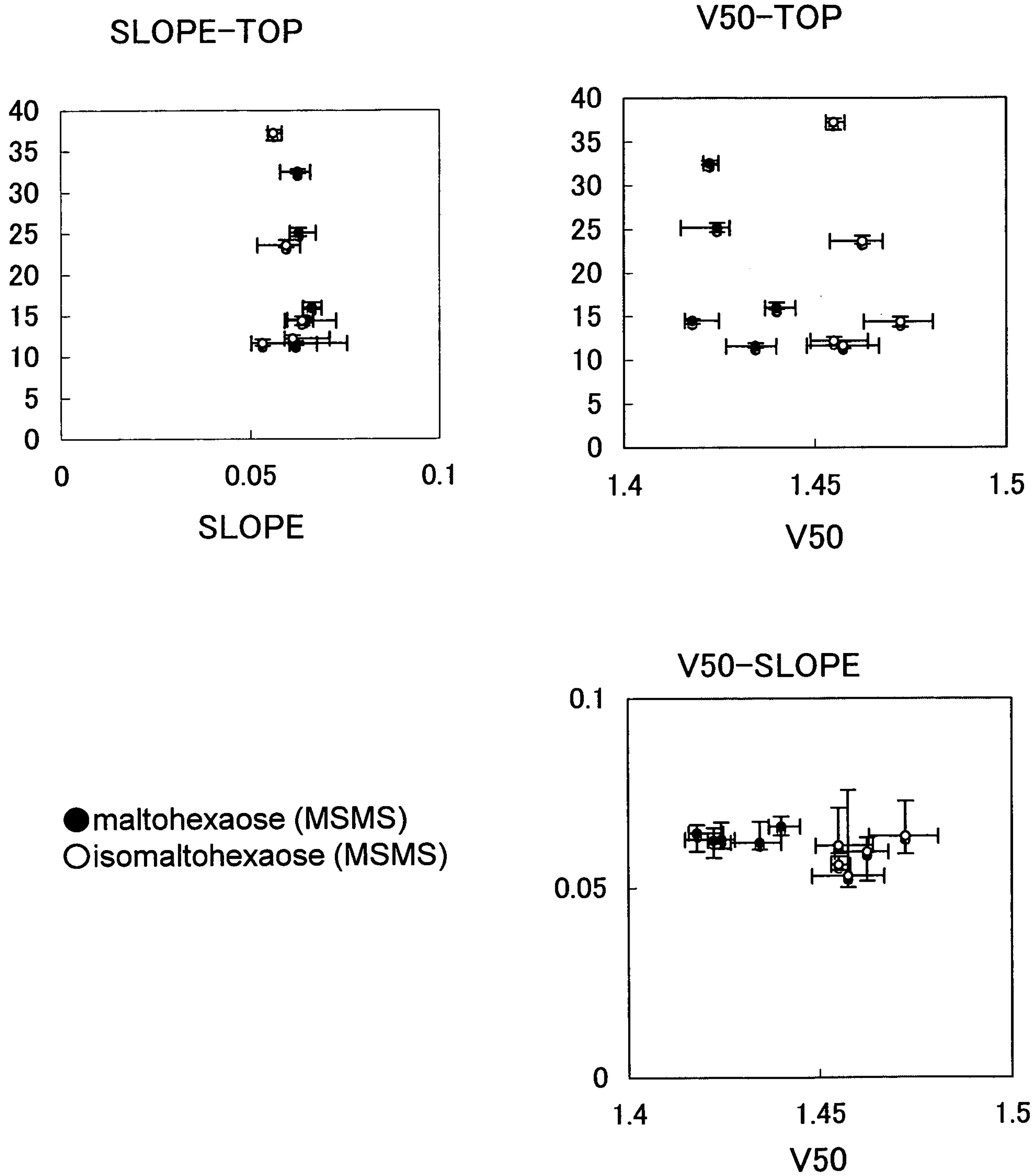
SLOPE-TOP
40
35
30
25
20
15
10
5
0
0
0.05
0.1
SLOPE
V50-TOP
40
35
30
25
20
15
10
5
0
1.4
1.45
1.5
V50
V50-SLOPE
0.1
0.05
0
1.4
1.45
1.5
V50
●maltohexaose (MSMS)
○isomaltohexaose (MSMS)

Fig. 14

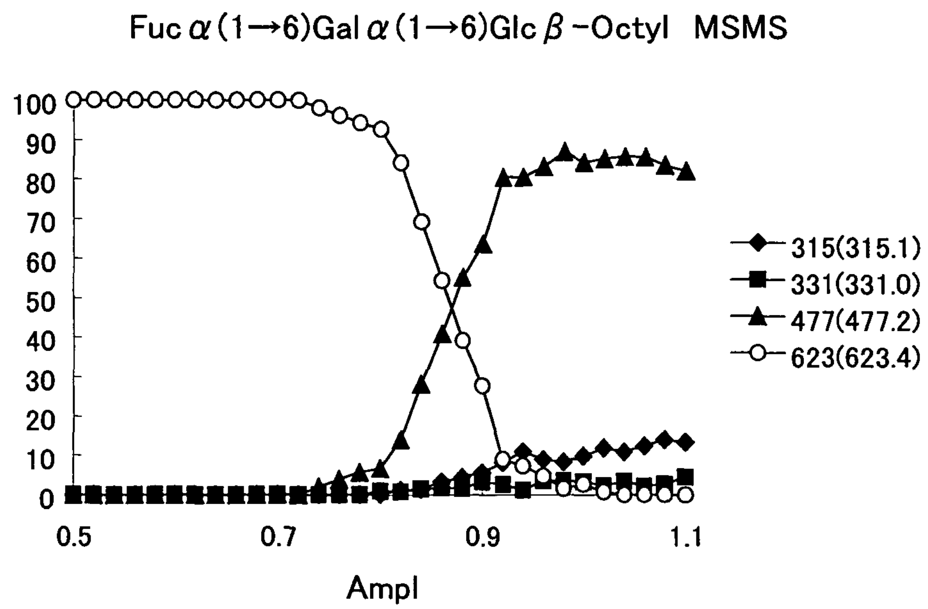
Fucα(1→6)Galα(1→6)Glcβ-Octyl MSMS
100
90
80
70
60
50
40
30
20
10
0
0.5
0.7
0.9
1.1
Ampl
315(315.1)
331(331.0)
477(477.2)
623(623.4)

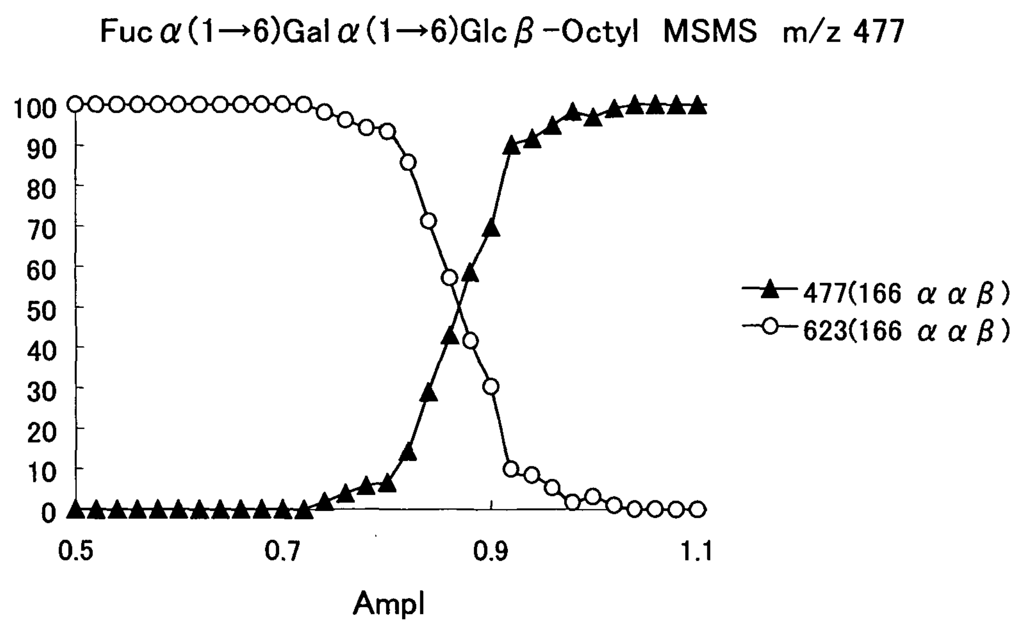
Fucα(1→6)Galα(1→6)Glcβ-Octyl MSMS m/z 477
100
90
80
70
60
50
40
30
20
10
0
0.5
0.7
0.9
1.1
Ampl
477(166 ααβ)
623(166 ααβ)

Fig.15

MS$^2$ data of Fuc$\alpha$1-6Gal$\alpha$1-6Glc$\beta$-Octyl

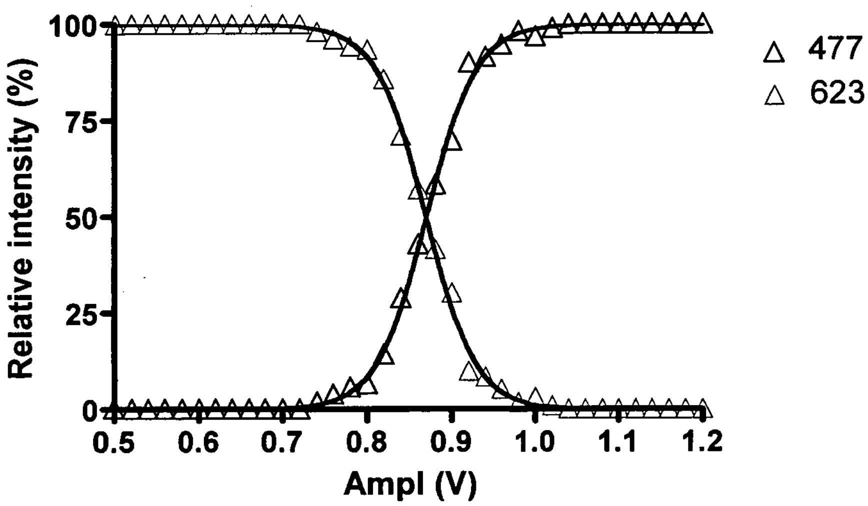


| Best-fit values | 477 |
|---|---|
| BOTTOM | 0 |
| TOP | 100 |
| V50 | 0.8694 |
| SLOPE | 0.02937 |

Fig.16

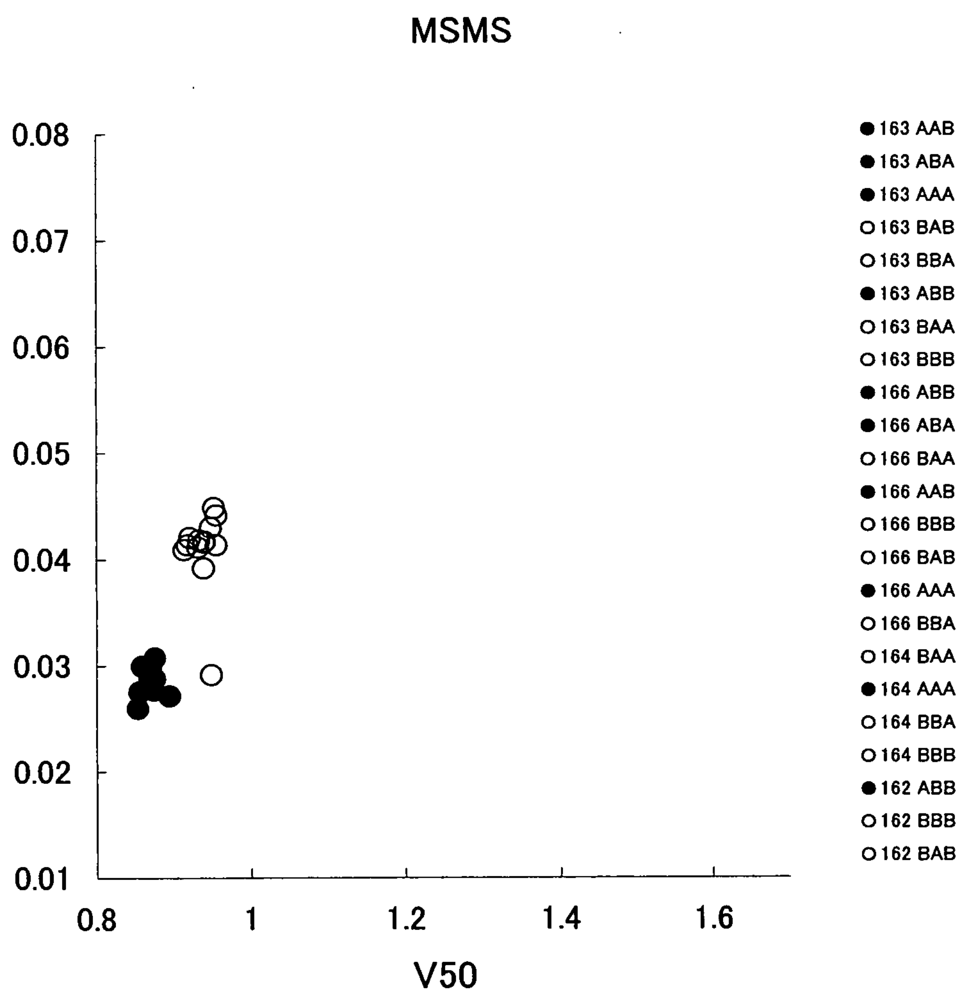
MSMS
0.08
0.07
0.06
0.05
0.04
0.03
0.02
0.01
0.8
1
1.2
1.4
1.6
V50
163 AAB
163 ABA
163 AAA
163 BAB
163 BBA
163 ABB
163 BAA
163 BBB
166 ABB
166 ABA
166 BAA
166 AAB
166 BBB
166 BAB
166 AAA
166 BBA
164 BAA
164 AAA
164 BBA
164 BBB
162 ABB
162 BBB
162 BAB

Fig.17

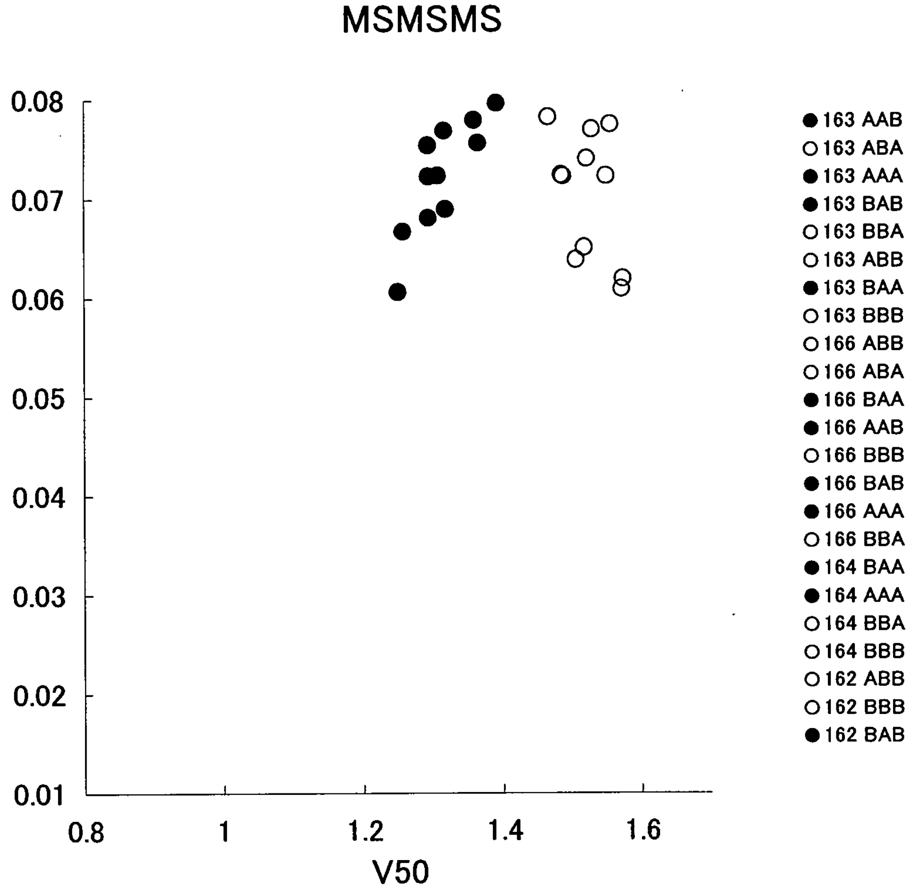
MSMSMS
0.08
0.07
0.06
0.05
0.04
0.03
0.02
0.01
0.8
1
1.2
1.4
1.6
V50
163 AAB
163 ABA
163 AAA
163 BAB
163 BBA
163 ABB
163 BAA
163 BBB
166 ABB
166 ABA
166 BAA
166 AAB
166 BBB
166 BAB
166 AAA
166 BBA
164 BAA
164 AAA
164 BBA
164 BBB
162 ABB
162 BBB
162 BAB

Fig.18

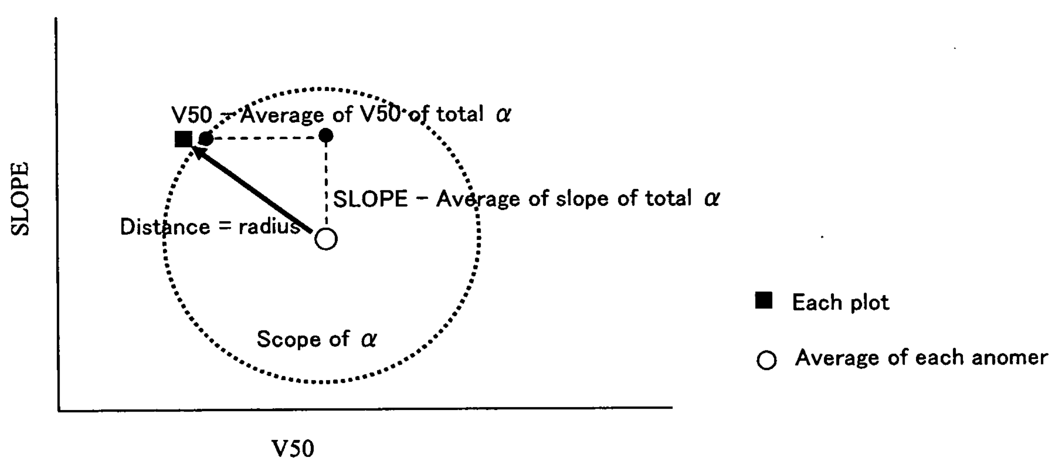
V50 – Average of V50 of total α
SLOPE – Average of slope of total α
Distance = radius
Scope of α
SLOPE
V50
Each plot
Average of each anomer

Fig.19

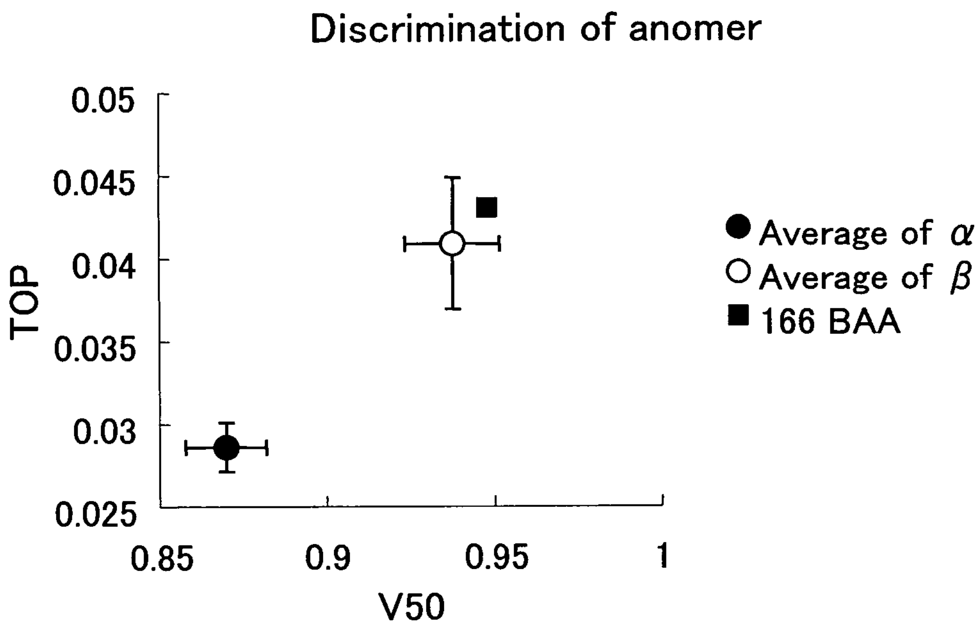
Discrimination of anomer
0.05
0.045
0.04
0.035
0.03
0.025
TOP
0.85
0.9
0.95
1
V50
Average of α
Average of β
166 BAA

Fig.20

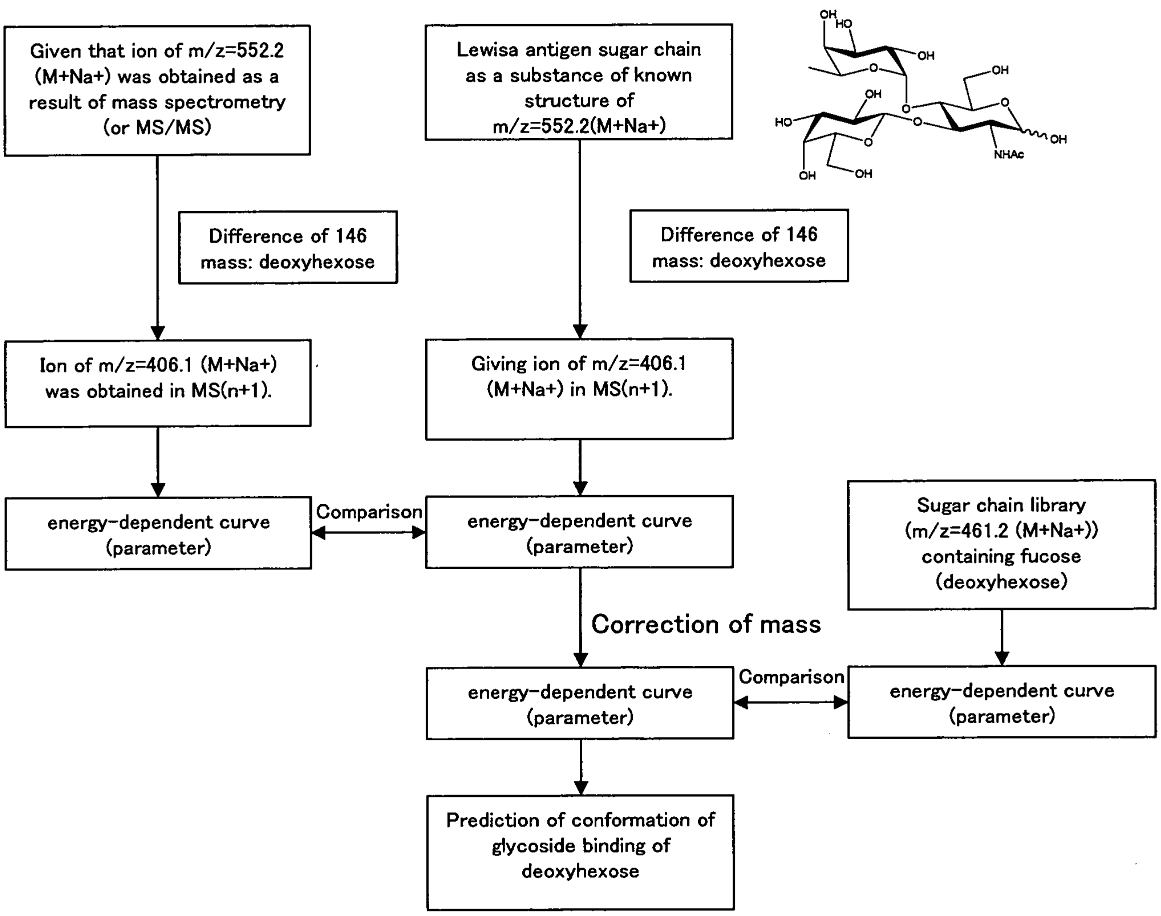
Given that ion of m/z=552.2 (M+Na+) was obtained as a result of mass spectrometry (or MS/MS)
Lewisa antigen sugar chain as a substance of known structure of m/z=552.2(M+Na+)
Difference of 146 mass: deoxyhexose
Difference of 146 mass: deoxyhexose
Ion of m/z=406.1 (M+Na+) was obtained in MS(n+1).
Giving ion of m/z=406.1 (M+Na+) in MS(n+1).
energy-dependent curve (parameter)
Comparison
energy-dependent curve (parameter)
Sugar chain library (m/z=461.2 (M+Na+)) containing fucose (deoxyhexose)
Correction of mass
energy-dependent curve (parameter)
Comparison
energy-dependent curve (parameter)
Prediction of conformation of glycoside binding of deoxyhexose

Fig.21

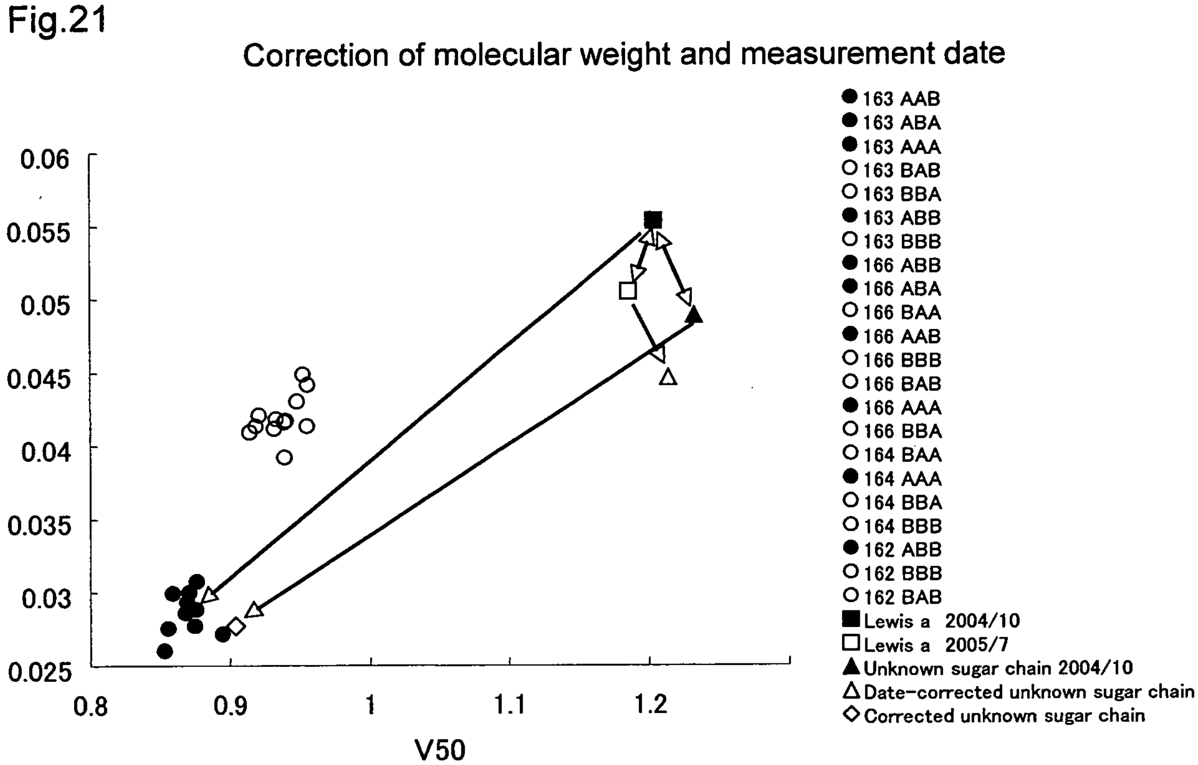
Correction of molecular weight and measurement date
0.06
0.055
0.05
0.045
0.04
0.035
0.03
0.025
0.8
0.9
1
1.1
1.2
V50
163 AAB
163 ABA
163 AAA
163 BAB
163 BBA
163 ABB
163 BBB
166 ABB
166 ABA
166 BAA
166 AAB
166 BBB
166 BAB
166 AAA
166 BBA
164 BAA
164 AAA
164 BBA
164 BBB
162 ABB
162 BBB
162 BAB
Lewis a 2004/10
Lewis a 2005/7
Unknown sugar chain 2004/10
Date-corrected unknown sugar chain
Corrected unknown sugar chain

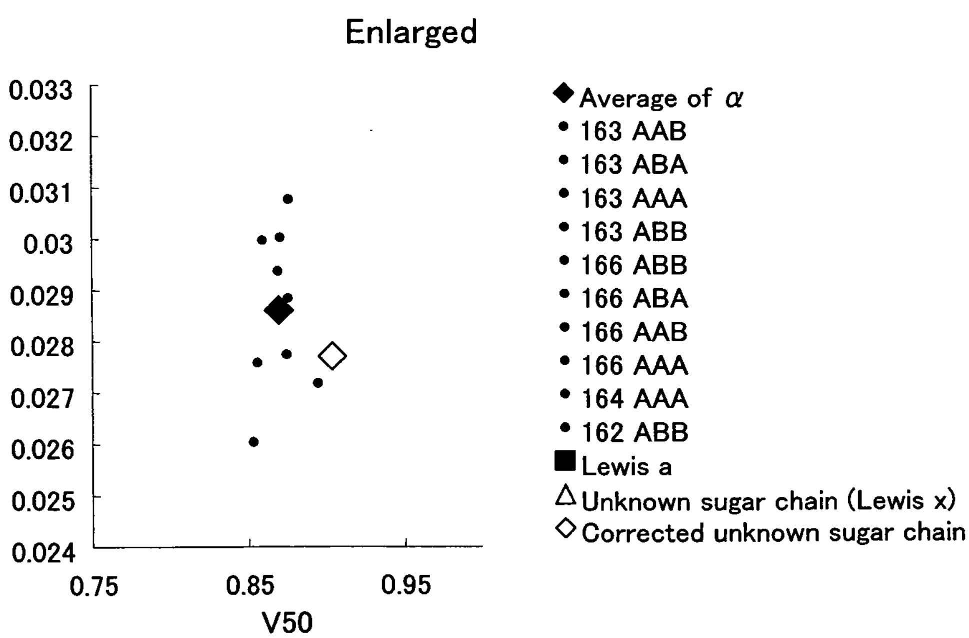
Enlarged
0.033
0.032
0.031
0.03
0.029
0.028
0.027
0.026
0.025
0.024
0.75
0.85
0.95
V50
Average of α
163 AAB
163 ABA
163 AAA
163 ABB
166 ABB
166 ABA
166 AAB
166 AAA
164 AAA
162 ABB
Lewis a
Unknown sugar chain (Lewis x)
Corrected unknown sugar chain

Fig.22

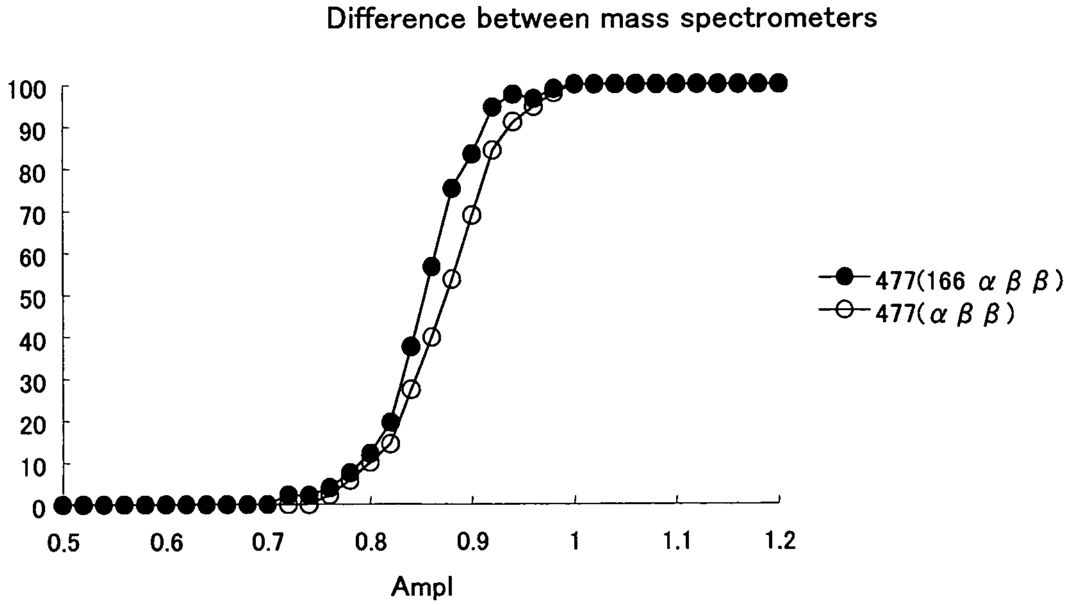


| First machine | |
|---|---|
| Best-fit values | m/z 477 |
| BOTTOM | 0 |
| TOP | 100 |
| V50 | 0.8533 |
| SLOPE | 0.02604 |

| Second machine | |
|---|---|
| Best-fit values | m/z 477 |
| BOTTOM | 0 |
| TOP | 100 |
| V50 | 0.873 |
| SLOPE | 0.03069 |

Corrected values

| | V50 | SLOPE |
|---|---|---|
| Not-corrected V50 | 0.9585 | 0.04686 |
| Corrected V50 | 0.9368706 | 0.03976 |
| Same sample, First machine, V50 | 0.9187 | 0.04136 |

# Fig.23

## A : Gal $\alpha$ 1-3Gal $\alpha$ -OMe

MSMS MSMSMS

0.87V

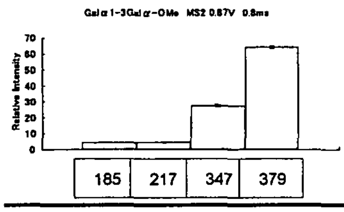


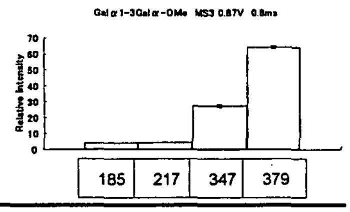


| Ampl | Ave.MS2(A) | Ave.MS3(B) | Difference (B-A) | Difference (absolute) |
|---|---|---|---|---|
| 185 | 4.291673279 | 4.205637254 | -0.086036025 | 0.086036025 |
| 217 | 4.47907401 | 4.415742707 | -0.063331304 | 0.063331304 |
| 347 | 27.2805908 | 27.21399068 | -0.06660012 | 0.06660012 |
| 379 | 63.94866191 | 64.16462936 | 0.215967449 | 0.215967449 |

0.91V

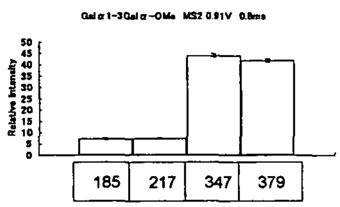


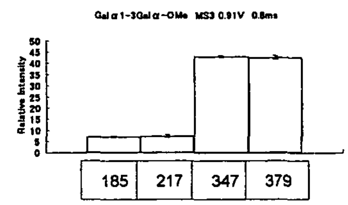


| Ampl | Ave.MS2(A) | Ave.MS3(B) | Difference (B-A) | Difference (absolute) |
|---|---|---|---|---|
| 185 | 7.23485707 | 7.165323937 | -0.069533133 | 0.069533133 |
| 217 | 7.344049074 | 7.435078365 | 0.091029291 | 0.091029291 |
| 347 | 43.86652455 | 42.97414055 | -0.892384001 | 0.892384001 |
| 379 | 41.55456931 | 42.42545715 | 0.870887843 | 0.870887843 |

0.96V

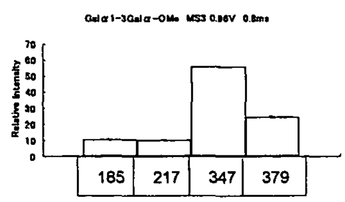


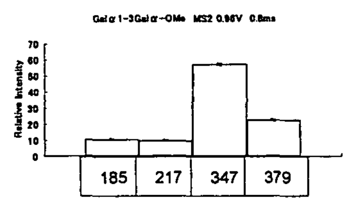


| Ampl | Ave.MS2(A) | Ave.MS3(B) | Difference (B-A) | Difference (absolute) |
|---|---|---|---|---|
| 185 | 10.50179111 | 10.26250365 | -0.239287451 | 0.239287451 |
| 217 | 9.685511916 | 9.877910155 | 0.192398239 | 0.192398239 |
| 347 | 57.25303185 | 55.64465285 | -1.608378994 | 1.608378994 |
| 379 | 22.55966513 | 24.21493334 | 1.655268206 | 1.655268206 |

# Fig.24

## B : Gal $\alpha$ 1-3Gal $\beta$ -OMe

MSMS | MSMSMS

0.90V

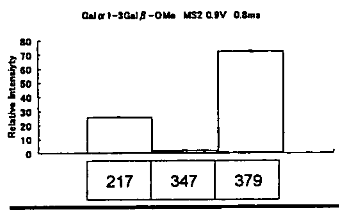


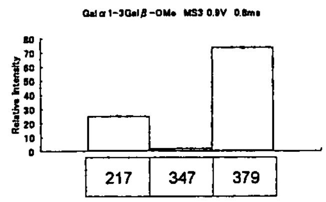


| Ampl | Ave.MS2(A) | Ave.MS3(B) | Difference (B-A) | Difference (absolute) |
|---|---|---|---|---|
| 217 | 25.73078656 | 24.73175795 | -0.999028613 | 0.999028613 |
| 347 | 1.988907127 | 2.010371004 | 0.021463877 | 0.021463877 |
| 379 | 72.28030631 | 73.25787105 | 0.977564736 | 0.977564736 |

0.98V

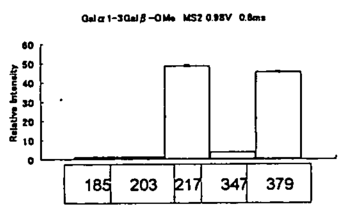


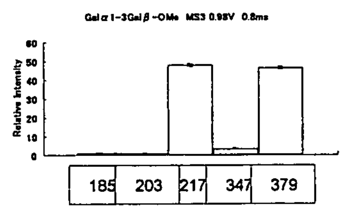


| Ampl | Ave.MS2(A) | Ave.MS3(B) | Difference (B-A) | Difference (absolute) |
|---|---|---|---|---|
| 185 | 1.16006403 | 1.177220927 | 0.017156897 | 0.017156897 |
| 203 | 1.184955094 | 1.034916676 | -0.150038418 | 0.150038418 |
| 217 | 48.55528189 | 47.92534649 | -0.629935397 | 0.629935397 |
| 347 | 3.707426535 | 3.467802669 | -0.239623866 | 0.239623866 |
| 379 | 45.39227246 | 46.39471324 | 1.002440784 | 1.002440784 |

1.04V

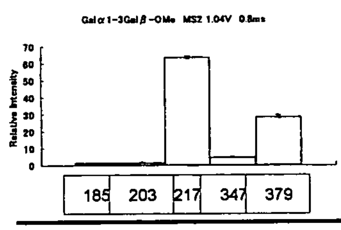


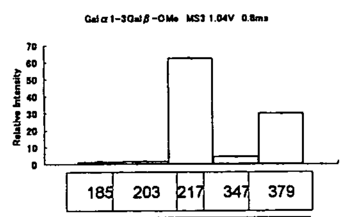


| Ampl | Ave.MS2(A) | Ave.MS3(B) | Difference (B-A) | Difference (absolute) |
|---|---|---|---|---|
| 185 | 1.412678377 | 1.496429438 | 0.083751061 | 0.083751061 |
| 203 | 1.553460169 | 1.66180761 | 0.108347441 | 0.108347441 |
| 217 | 63.57790073 | 62.45316586 | -1.124734868 | 1.124734868 |
| 347 | 4.768716137 | 4.464844189 | -0.303871947 | 0.303871947 |
| 379 | 28.68724459 | 29.92375291 | 1.236508314 | 1.236508314 |

# Fig.25

## A:B = 1:1

MSMS | MSMSMS

### 0.91V

A:B = 1:1 MSMS 0.91V

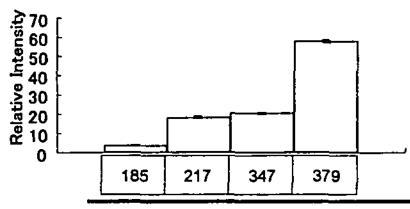


A:B = 1:1 MSMSMS 0.91V

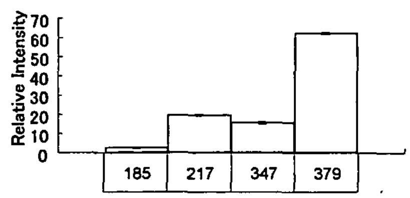


| Ampl | Ave.MS2(A) | Ave.MS3(B) | Difference (B-A) | Difference (absolute) |
|---|---|---|---|---|
| 185 | 3.484124945 | 2.669347427 | -0.814777518 | 0.814777518 |
| 217 | 18.30835749 | 19.51647481 | 1.20811732 | 1.20811732 |
| 347 | 20.46888782 | 15.53026173 | -4.938626087 | 4.938626087 |
| 379 | 57.73862974 | 62.28391603 | 4.545286284 | 4.545286284 |

### 0.98V

A:B = 1:1 MSMS 0.98V

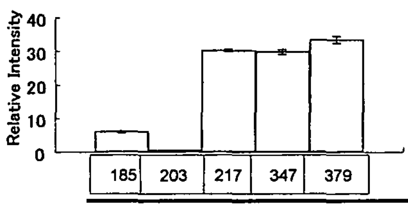


A:B = 1:1 MSMSMS 0.98V

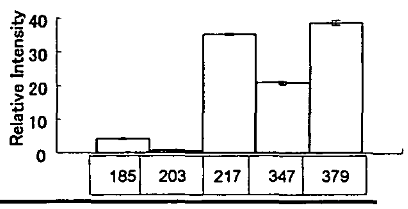


| Ampl | Ave.MS2(A) | Ave.MS3(B) | Difference (B-A) | Difference (absolute) |
|---|---|---|---|---|
| 185 | 5.985056558 | 4.214056227 | -1.771000331 | 1.771000331 |
| 203 | 0.638853003 | 0.811426768 | 0.172573765 | 0.172573765 |
| 217 | 30.1725796 | 35.33544046 | 5.162860865 | 5.162860865 |
| 347 | 29.80854413 | 20.88359805 | -8.924946081 | 8.924946081 |
| 379 | 33.39496671 | 38.75547849 | 5.360511783 | 5.360511783 |

### 1.05V

A:B = 1:1 MSMS 1.05V

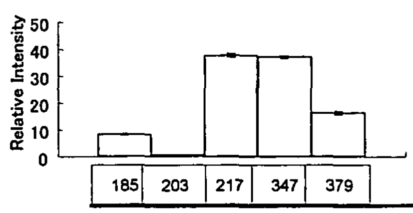


A:B = 1:1 MSMSMS 1.05V

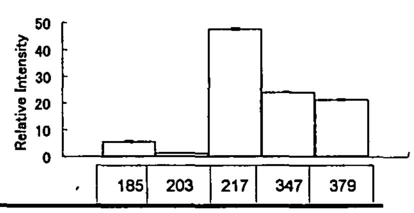


| Ampl | Ave.MS2(A) | Ave.MS3(B) | Difference (B-A) | Difference (absolute) |
|---|---|---|---|---|
| 185 | 8.229682068 | 5.636868146 | -2.592813923 | 2.592813923 |
| 203 | 0.779632641 | 1.259585555 | 0.479952914 | 0.479952914 |
| 217 | 37.66115564 | 47.68850545 | 10.02734981 | 10.02734981 |
| 347 | 37.05919324 | 24.09569507 | -12.96349817 | 12.96349817 |
| 379 | 16.27033641 | 21.31934578 | 5.049009372 | 5.049009372 |

# Fig.26

## A:B = 1:9

MSMS

MSMSMS

0.88V

A:B = 1:9 MSMS 0.88V

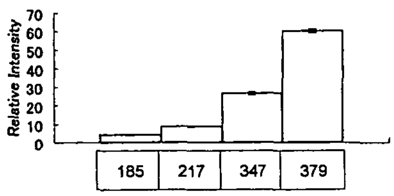


A:B = 1:9 MSMSMS 0.88V

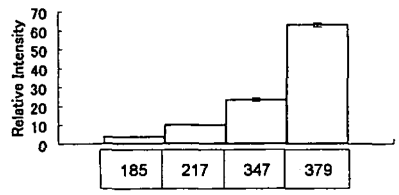


| Ampl | Ave.MS2(A) | Ave.MS3(B) | Difference (B-A) | Difference (absolute) |
|---|---|---|---|---|
| 185 | 4.268427092 | 3.697410827 | -0.571016265 | 0.571016265 |
| 217 | 8.875992966 | 10.05705734 | 1.181064371 | 1.181064371 |
| 347 | 26.67459825 | 23.31471616 | -3.359882094 | 3.359882094 |
| 379 | 60.18098169 | 62.93081568 | 2.749833988 | 2.749833988 |

0.93V

A:B = 1:9 MSMS 0.93V

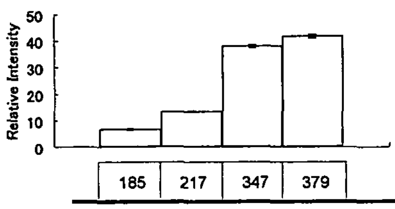


A:B = 1:9 MSMSMS 0.93V

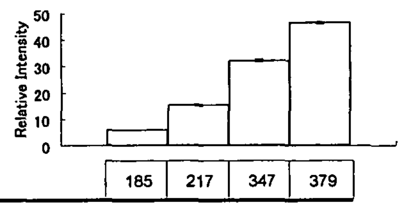


| Ampl | Ave.MS2(A) | Ave.MS3(B) | Difference (B-A) | Difference (absolute) |
|---|---|---|---|---|
| 185 | 6.699756906 | 5.919388937 | -0.780367968 | 0.780367968 |
| 217 | 13.41044777 | 15.33931335 | 1.928865582 | 1.928865582 |
| 347 | 38.06541428 | 32.18630801 | -5.879106271 | 5.879106271 |
| 379 | 41.82438105 | 46.5549897 | 4.730608657 | 4.730608657 |

0.99V

A:B = 1:9 MSMS 0.99V

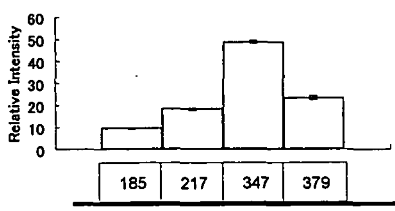


A:B = 1:9 MSMSMS 0.99V

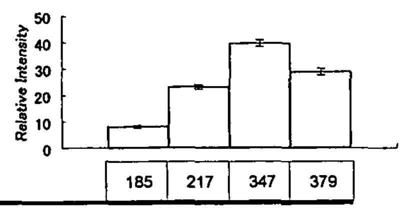


| Ampl | Ave.MS2(A) | Ave.MS3(B) | Difference (B-A) | Difference (absolute) |
|---|---|---|---|---|
| 185 | 9.659541653 | 8.154820648 | -1.504721005 | 1.504721005 |
| 217 | 18.25663222 | 23.17098712 | 4.9143549 | 4.9143549 |
| 347 | 48.72679769 | 39.74600932 | -8.980788363 | 8.980788363 |
| 379 | 23.35702844 | 28.92818291 | 5.571154468 | 5.571154468 |

## INTERNATIONAL SEARCH REPORT

International application No.
PCT/JP2005/018134

A. CLASSIFICATION OF SUBJECT MATTER
*G01N27/62*(2006.01), *G01N33/483*(2006.01)

According to International Patent Classification (IPC) or to both national classification and IPC

B. FIELDS SEARCHED

Minimum documentation searched (classification system followed by classification symbols)
G01N27/62-G01N27/70, H01J49/00-H01J49/48

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y | Osamu KANIE, "Shitsuryo Bunsekiho ni yoru Teiryoteki Kairetsu Hanno Kaiseki", Polymer Preprints, Japan, Vol.53, No.2, 01 September, 2004 (01.09.04), pages 5378 to 5379 | 1,8,9,11<br>7,10,12,13 |
| Y | JP 2004-191077 A (Hitachi, Ltd.), 08 July, 2004 (08.07.04), Claim 15; Par. Nos. [0002], [0025]; Fig. 9 & US 2004/0111228 A1 & EP 1429145 A1 | 7,10,13 |
| Y | JP 2003-507874 A (University of New Hampshire), 25 February, 2003 (25.02.03), Par. No. [0003] & WO 01/15201 A2 | 12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>16 December, 2005 (16.12.05) | Date of mailing of the international search report<br>27 December, 2005 (27.12.05) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2005/018134

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-245699 A (Hitachi, Ltd.), 02 September, 2004 (02.09.04), Par. No. [0040] & US 2004/0181347 A1 & EP 1447833 A2 | 14 |
| Y | JP 2005-520176 A (HK Pharmaceuticals, Inc.), 07 July, 2005 (07.07.05), Par. No. [0094] & WO 2003/092581 A2 | 14 |

Form PCT/ISA/210 (continuation of second sheet) (April 2005)

**INTERNATIONAL SEARCH REPORT**

International application No.
PCT/JP2005/018134

**Box No. II Observations where certain claims were found unsearchable (Continuation of item 2 of first sheet)**

This international search report has not been established in respect of certain claims under Article 17(2)(a) for the following reasons:

1. [X] Claims Nos.: 2-6
because they relate to subject matter not required to be searched by this Authority, namely:
claims 2-6 are directed to mere presentation of information.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See attachment (extra sheet).

1. [X] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[X] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

**INTERNATIONAL SEARCH REPORT**

International application No.
PCT/JP2005/018134

Continuation of Box No.III of continuation of first sheet(2)

The matter common to the invention of claims 1, 8 and 9, the invention of claims 7, 10 and 13, the invention of claim 11, the invention of claim 12 and the invention of claim 14 is use of a CID energy-dependent curve.

However, search has revealed that this common matter is not novel as it is disclosed in the reference (1): Osamu KANIE, "Shitsuryo Bunsekiho ni yoru Teiryoteki Kairetsu Hanno Kaiseki", Polymer Preprints, Japan, Vol.53, No.2, 01 September, 2004 (01.09.04), pages 5378 to 5379

Consequently, the above common matter falls within the category of prior art and hence the common matter is not a special technical feature within the meaning of PCT Rule 13.2, second sentence.

Therefore, there is no matter common to all of the invention of claims 1, 8 and 9, the invention of claims 7, 10 and 13, the invention of claim 11, the invention of claim 12 and the invention of claim 14.

As there exists no other common matter which can be considered as a special technical feature within the meaning of PCT Rule 13.2, second sentence, no technical relationship within the meaning of PCT Rule 13 can be found among the different inventions.

Therefore, it is apparent that the invention of claims 1, 8 and 9, the invention of claims 7, 10 and 13, the invention of claim 11, the invention of claim 12 and the invention of claim 14 do not satisfy the requirement of unity of invention.

With respect to claims 2-6, it has been found that they relate to a subject matter for which no international search is required (see Box No. II). Even if they were a subject matter requiring international search, the claims 2-6 are directed to an invention whose special technical feature is identical with that of the invention of claims 7, 10 and 13, and accordingly there is no influence upon judgment of the unity of invention, especially the number of inventions.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004009523 W
- JP 4009523 W


### Non-patent literature cited in the description


- **ROYLE, L. et al.** *Anal. Biochem.,* 2002, vol. 304, 70-90
- **CHAPLIN, M. F. et al.** Carbohydrate Analysis, A Practical Approach. IRL Press, 1994
- **CHAPLIN, M.F et al.** Carbohydrate Analysis, A Practical Approach. IRL Press, 1994
- **CHAPLIN, M. F et al.** Carbohydrate Analysis, A Practical Approach. IRL Press, 1994
- **DELL, A.** *Adv. Carbohydr. Chem. Biochem.,* 1987, vol. 45, 19-72
- **FARETTO, D. et al.** *Mass Spectrom.,* 1991, vol. 5, 240-244
- **KURONO, S. et al.** *J. Mass Spectrom.,* 1998, vol. 33, 35-44
- **VISEUX. N. et al.** *Anal. Chem.,* 1998, vol. 70, 4951-4959
- **KAMEYAMA, A. et al.** *Anal. Chem.,* 2005, vol. 77, 4719-4725
- **FAENGMARK, I. et al.** *Anal. Chem.,* 1999, vol. 71, 1105-1110